# EUROPEAN PATENT APPLICATION

(11) **EP 2 390 325 A1**
(43) Date of publication of application: **30.11.2011**
(21) Application number: 10733486.4
(22) Date of filing: 20.01.2010
(51) Int. Cl.: C12N 15/09, C12N 1/15, C12N 1/19, C12N 1/21, C12N 5/10, C12N 9/10, C12P 19/00

(54) **FLAVONOID-3-GLUCURONYLTRANSFERASE AND POLYNUCLEOTIDE ENCODING SAME**

(30) Priority: 21.01.2009 JP 2009011065
(71) Applicant: Suntory Holdings Limited, Kita-ku, Osaka-shi Osaka 530-8203 (JP)
(72) Inventor: ONO Eiichiro, Mishima-gun Osaka 618-8503 (JP); FUKUI Yuko, Mishima-gun Osaka 618-8503 (JP); NAKAYAMA Toru, Sendai-shi Miyagi 980-8579 (JP)
(74) Representative: Vossius & Partner
(86) International application number: PCT/JP2010/050629
(87) International publication number: WO 2010/084879

(57) **Abstract**

The invention provides an enzyme (F3GAT) which can transfer glucuronic acid to position-3 in a flavonoid, particularly a flavonol; a polynucleotide encoding the enzyme; a vector comprising the polynucleotide; a transformant produced using the vector; and so on. The polynucleotide in accordance with the invention comprises a polynucleotide comprising the nucleotide sequence represented by SEQ ID NO:3 or a polynucleotide encoding a protein comprising the amino acid sequence represented by SEQ ID NO:4.

SEQUENCE LISTING

<110>  Suntory Holdings Limited

<120>  Flavonoid 3-O-glucuronic acid transferase and polynucleotide
       encoding the same

<130>  T2369 EP S3

<140>  EP 10 73 3486.4
<141>  2010-01-20

<150>  JP 2009-011065
<151>  2009-01-21

<160>  14

<170>  PatentIn version 3.4

<210>  1
<211>  30
<212>  DNA
<213>  Artificial

<220>
<223>  synthetic DNA

<400>  1
cacccatatg actaccaccg ccagctccat                                        30


<210>  2
<211>  29
<212>  DNA
<213>  Artificial

<220>
<223>  synthetic DNA

<400>  2
agatctctac ttattggtgt ccaaaggta                                         29


<210>  3
<211>  1380
<212>  DNA
<213>  Vitis vinifera


<220>
<221>  CDS
<222>  (1)..(1380)

<400>  3
atg act acc acc gcc agc tcc atg gac agg cat gtt gca gta tta ggg       48
Met Thr Thr Thr Ala Ser Ser Met Asp Arg His Val Ala Val Leu Gly
1               5                   10                  15

ttc ccc acc cat aca gct acc ctc tta aaa ctc ctg cgc aga cta gca       96
Phe Pro Thr His Thr Ala Thr Leu Leu Lys Leu Leu Arg Arg Leu Ala
            20                  25                  30

tct gcc gca ccc acc acc atc ttc tcc ttc ttc aac act ccc aag gcc      144
Ser Ala Ala Pro Thr Thr Ile Phe Ser Phe Phe Asn Thr Pro Lys Ala
        35                  40                  45

aac agc tcc atc tcc tct gct caa agt ccc cac ggt att cac aat cta 192
Asn Ser Ser Ile Ser Ser Ala Gln Ser Pro His Gly Ile His Asn Leu
    50                  55                  60

aga gtt tat gat gta gca gac ggc gtg cca gag gac ctt gtg ctt tca 240
Arg Val Tyr Asp Val Ala Asp Gly Val Pro Glu Asp Leu Val Leu Ser
65                  70                  75                  80

gca aac ccc ctg gca cgc atc gag atg ttc ttg aag gcg acg cct ggg 288
Ala Asn Pro Leu Ala Arg Ile Glu Met Phe Leu Lys Ala Thr Pro Gly
                85                  90                  95

aac ttt cgt gat gct ttg gaa gtg gct gag aag gac att gga agg aag 336
Asn Phe Arg Asp Ala Leu Glu Val Ala Glu Lys Asp Ile Gly Arg Lys
            100                 105                 110

ata agt tgc ctg gtg agt gat gtc ttt ttg tgg ttc act gct gat atg 384
Ile Ser Cys Leu Val Ser Asp Val Phe Leu Trp Phe Thr Ala Asp Met
        115                 120                 125

gct gag gaa atg ggg gtt ccg tgg gtg gca att agg act gct gcg ctt 432
Ala Glu Glu Met Gly Val Pro Trp Val Ala Ile Arg Thr Ala Ala Leu
    130                 135                 140

tac tca ctc tcc gtc cac att tat act gat gct atc cgg gaa gca gtg 480
Tyr Ser Leu Ser Val His Ile Tyr Thr Asp Ala Ile Arg Glu Ala Val
145                 150                 155                 160

gga gtt gcg ggg cag gtg caa gac caa acc ctt gat ttc atc cca gga 528
Gly Val Ala Gly Gln Val Gln Asp Gln Thr Leu Asp Phe Ile Pro Gly
                165                 170                 175

ttc tca gca ata aag gtt gaa gac cta cct gaa gga atg gtt ttc ggg 576
Phe Ser Ala Ile Lys Val Glu Asp Leu Pro Glu Gly Met Val Phe Gly
            180                 185                 190

gac aca gaa tct ccc ttc gca tgc atg ttg cat aaa atg ggg ctc atg 624
Asp Thr Glu Ser Pro Phe Ala Cys Met Leu His Lys Met Gly Leu Met
        195                 200                 205

ctg cca cga gca acc att gtt gcc aca aat tcc ttt gaa gaa cta gag 672
Leu Pro Arg Ala Thr Ile Val Ala Thr Asn Ser Phe Glu Glu Leu Glu
    210                 215                 220

ccg acc att gtc aca aat gat ctc aag tcc aag ctc caa aaa gtt ctt 720
Pro Thr Ile Val Thr Asn Asp Leu Lys Ser Lys Leu Gln Lys Val Leu
225                 230                 235                 240

act gtt ggt cct ttt gac cta tct tca cca cca ccg ctg ata ttg gac 768
Thr Val Gly Pro Phe Asp Leu Ser Ser Pro Pro Pro Leu Ile Leu Asp
                245                 250                 255

gcc agt ggc tgc ctg cca tgg ttg gac aat aaa aaa gaa gcg tca gtg 816
Ala Ser Gly Cys Leu Pro Trp Leu Asp Asn Lys Lys Glu Ala Ser Val
            260                 265                 270

gca tat gtt agt ttt gga agc ata gca aca cca cca ccc aac gag att 864
Ala Tyr Val Ser Phe Gly Ser Ile Ala Thr Pro Pro Pro Asn Glu Ile
        275                 280                 285

gta gca ttg gca gaa gcc cta gaa gca act ggg ata ccg ttt ctt tgg 912
Val Ala Leu Ala Glu Ala Leu Glu Ala Thr Gly Ile Pro Phe Leu Trp
    290                 295                 300

tct ctt agg gag cat gca atg aac aat tta cca aaa gga ttt cta gag      960
Ser Leu Arg Glu His Ala Met Asn Asn Leu Pro Lys Gly Phe Leu Glu
305                 310                 315                 320

agg acg acc gct cat gga aaa gta gtt tcg tgg gca ccc cag cct cag      1008
Arg Thr Thr Ala His Gly Lys Val Val Ser Trp Ala Pro Gln Pro Gln
                325                 330                 335

gtc tta gca cat gcc tca gtt gca gtg ttt att act cat agc ggt tgg      1056
Val Leu Ala His Ala Ser Val Ala Val Phe Ile Thr His Ser Gly Trp
            340                 345                 350

aac tcg gtg act gag agt ata gtt ggt ggt gtg ccc atg atc tgc agg      1104
Asn Ser Val Thr Glu Ser Ile Val Gly Gly Val Pro Met Ile Cys Arg
        355                 360                 365

cca ttc ttt gga gat caa cgt ctt aac agg cgg atg gta cag gat gta      1152
Pro Phe Phe Gly Asp Gln Arg Leu Asn Arg Arg Met Val Gln Asp Val
    370                 375                 380

tgg ggg att ggt ata gga gtt gag gga ggg atc ctc aca aaa agg gga      1200
Trp Gly Ile Gly Ile Gly Val Glu Gly Gly Ile Leu Thr Lys Arg Gly
385                 390                 395                 400

gta atg agt gct ttg gga cta att ttg tcc cat gaa ggg aag aaa atg      1248
Val Met Ser Ala Leu Gly Leu Ile Leu Ser His Glu Gly Lys Lys Met
                405                 410                 415

aga gag aaa att ggg gtc cta aaa gag ctt gct aga agg gct gtt gaa      1296
Arg Glu Lys Ile Gly Val Leu Lys Glu Leu Ala Arg Arg Ala Val Glu
            420                 425                 430

cca aac ggg agc tca act caa aat tta ggt cat ttg ttg gag gta atc      1344
Pro Asn Gly Ser Ser Thr Gln Asn Leu Gly His Leu Leu Glu Val Ile
        435                 440                 445

aca aca tct aag tta cct ttg gac acc aat aag tag                      1380
Thr Thr Ser Lys Leu Pro Leu Asp Thr Asn Lys
    450                 455


<210>  4
<211>  459
<212>  PRT
<213>  Vitis vinifera

<400>  4

Met Thr Thr Thr Ala Ser Ser Met Asp Arg His Val Ala Val Leu Gly
1               5                   10                  15


Phe Pro Thr His Thr Ala Thr Leu Leu Lys Leu Leu Arg Arg Leu Ala
            20                  25                  30


Ser Ala Ala Pro Thr Thr Ile Phe Ser Phe Phe Asn Thr Pro Lys Ala
        35                  40                  45


Asn Ser Ser Ile Ser Ser Ala Gln Ser Pro His Gly Ile His Asn Leu
    50                  55                  60


Arg Val Tyr Asp Val Ala Asp Gly Val Pro Glu Asp Leu Val Leu Ser

65                  70                  75                  80

Ala Asn Pro Leu Ala Arg Ile Glu Met Phe Leu Lys Ala Thr Pro Gly
                85                  90                  95

Asn Phe Arg Asp Ala Leu Glu Val Ala Glu Lys Asp Ile Gly Arg Lys
            100                 105                 110

Ile Ser Cys Leu Val Ser Asp Val Phe Leu Trp Phe Thr Ala Asp Met
        115                 120                 125

Ala Glu Glu Met Gly Val Pro Trp Val Ala Ile Arg Thr Ala Ala Leu
    130                 135                 140

Tyr Ser Leu Ser Val His Ile Tyr Thr Asp Ala Ile Arg Glu Ala Val
145                 150                 155                 160

Gly Val Ala Gly Gln Val Gln Asp Gln Thr Leu Asp Phe Ile Pro Gly
                165                 170                 175

Phe Ser Ala Ile Lys Val Glu Asp Leu Pro Glu Gly Met Val Phe Gly
            180                 185                 190

Asp Thr Glu Ser Pro Phe Ala Cys Met Leu His Lys Met Gly Leu Met
        195                 200                 205

Leu Pro Arg Ala Thr Ile Val Ala Thr Asn Ser Phe Glu Glu Leu Glu
    210                 215                 220

Pro Thr Ile Val Thr Asn Asp Leu Lys Ser Lys Leu Gln Lys Val Leu
225                 230                 235                 240

Thr Val Gly Pro Phe Asp Leu Ser Ser Pro Pro Pro Leu Ile Leu Asp
                245                 250                 255

Ala Ser Gly Cys Leu Pro Trp Leu Asp Asn Lys Lys Glu Ala Ser Val
            260                 265                 270

Ala Tyr Val Ser Phe Gly Ser Ile Ala Thr Pro Pro Pro Asn Glu Ile
        275                 280                 285

Val Ala Leu Ala Glu Ala Leu Glu Ala Thr Gly Ile Pro Phe Leu Trp
    290                 295                 300

Ser Leu Arg Glu His Ala Met Asn Asn Leu Pro Lys Gly Phe Leu Glu
305                 310                 315                 320

Arg Thr Thr Ala His Gly Lys Val Val Ser Trp Ala Pro Gln Pro Gln
                325                 330                 335

Val Leu Ala His Ala Ser Val Ala Val Phe Ile Thr His Ser Gly Trp
            340                 345                 350

Asn Ser Val Thr Glu Ser Ile Val Gly Gly Val Pro Met Ile Cys Arg
        355                 360                 365

Pro Phe Phe Gly Asp Gln Arg Leu Asn Arg Arg Met Val Gln Asp Val
    370                 375                 380

Trp Gly Ile Gly Ile Gly Val Glu Gly Gly Ile Leu Thr Lys Arg Gly
385                 390                 395                 400

Val Met Ser Ala Leu Gly Leu Ile Leu Ser His Glu Gly Lys Lys Met
                405                 410                 415

Arg Glu Lys Ile Gly Val Leu Lys Glu Leu Ala Arg Arg Ala Val Glu
            420                 425                 430

Pro Asn Gly Ser Ser Thr Gln Asn Leu Gly His Leu Leu Glu Val Ile
        435                 440                 445

Thr Thr Ser Lys Leu Pro Leu Asp Thr Asn Lys
    450                 455

<210>  5
<211>  21
<212>  DNA
<213>  Artificial

<220>
<223>  synthetic DNA

<400>  5
gctccatctc ctctgctcaa a                                                21

<210>  6
<211>  18
<212>  DNA
<213>  Artificial

<220>
<223>  synthetic DNA

<400>  6
gaaagcacaa ggtcctct                                                    18

<210>  7
<211>  18
<212>  DNA
<213>  Artificial

<220>
<223>  synthetic DNA

<400>  7
cccaccgccg gttatacc                                                    18


<210>  8
<211>  19
<212>  DNA
<213>  Artificial

<220>
<223>  synthetic DNA

<400>  8
cgaccgaggt gggttttct                                                   19


<210>  9
<211>  20
<212>  DNA
<213>  Artificial

<220>
<223>  synthetic DNA

<400>  9
ggttccctgg ttggcaattt                                                  20


<210>  10
<211>  19
<212>  DNA
<213>  Artificial

<220>
<223>  synthetic DNA

<400>  10
gcacccgccc cacaacctt                                                   19


<210>  11
<211>  21
<212>  DNA
<213>  Artificial

<220>
<223>  synthetic DNA

<400>  11
tccaggacaa ggaagggatt c                                                21


<210>  12
<211>  20
<212>  DNA
<213>  Artificial

<220>
<223>  synthetic DNA

<400>  12
gccatcctca agctgctttc                                                  20

<210>  13
<211>  21
<212>  DNA
<213>  Artificial

<220>
<223>  synthetic DNA

<400>  13
gggtggcaat ttggactgct g                                                21


<210>  14
<211>  20
<212>  DNA
<213>  Artificial

<220>
<223>  synthetic DNA

<400>  14
cagcagtcca aattgcaccc                                                  20

## Description

### TECHNICAL FIELD

The present invention relates to a flavonoid 3-O-glucuronosyltransferase, a polynucleotide encoding the enzyme, a vector comprising the polynucleotide, a transformant produced using the vector; and so on.

### BACKGROUND ART

Polyphenolic plant secondary metabolites including flavonoids and lignans have been extensively used as health foods. These metabolites undergo glucuronide conjugation *in vivo* by a glycosyltransferase called UGT. In general, glucuronides are excreted as urine and hence, their function has not hitherto been considered a subject for study. In recent years, the biological activities of morphine glucuronides or flavonoid glucuronides become known (cf., Non-Patent Literatures 1 and 2).

Vitis vinifera used as a raw material for wine is the major product of agricultural crops worldwide. Red wine is known to have useful functionality such as reduced risk of heart disease, antiobestic effects, etc. In recent years, resveratrol or a class of stilbens, as the main functional component of antiobestic or lifespan extension effects and a procianidin as a factor for the French paradox have been identified in red wine and has attracted attention as a functional food (cf., Non-Patent Literatures 3 - 9).

It is reported that quercetin-3-glucoside and quercetin-3-glucuronoside are accumulated in grapevine leaves as the glycosides of a flavonol which is one of flavonoids (cf., Non-Patent Literature 10). Furthermore, the accumulation of various flavonol glycosides in grape berries and wine is also reported in recent years (cf:, Non-Patent Literature 11). In particular, quercetin 3-glucuronoside is called miquelianin, which is known to be one of the main pharmaceutical ingredients of St. John's wort used as an antidepressant, and is shown to be absorbed from the intestinal tract through oral administration to reach the central nervous system. However, quercetin 3--rhamnoside has no such activity (cf , Non-Patent Literature 12). Furthermore, quercetin 3-glucuronoside is known to have an inhibitory effect on LDL degradation (cf., Non-Patent Literatures 13 and 14), an effect of decreasing intracellular oxidative stress induced by the attack of H₂O₂ (cf., Non-Patent Literature 15), an inhibitory effect on angiotensin-induced vascular smooth muscle cell hypertrophy (prevention of heart disease) (cf., Non-Patent Literature 16), and the like.

In view of these studies, enzymes that transfer glucuronic acid to flavonoids, especially flavonols at the position 3 are considered useful to produce functional flavonoids. Only the enzymes that transfer glucose, galactose, arabinose and rhamnose as sugar donors are known as flavonoid 3-glycosyltransferases. Enzymes that transfer glucuronic acid as a sugar donor and genes encoding the same are unknown (cf, Non-Patent Literatures 17-20).

Further in recent years, the genome sequence of grapevine (Pinot Noir cultivars) was decoded by The French-Italian Public Consortium and even 240 glycosyltransferases (UGT) genes were identified (cf, Non-Patent Literatures 21 and 22).

However, even after it was deduced from gene sequence information to be a glycosyltransferase gene, it was not easy to identify, by analogy, the type of sugar as a substrate or a substance as an acceptor based on the information. Moreover, the number of candidate genes is as extremely large as 240 as described above. For these reasons, it was extremely difficult to identify among these UGT genes flavonoid 3-O-glucuronosyltransferase (flavonoid 3-O-glucuronic acid transferase; F3GAT), which has not been hitherto reported either in the other living organisms.

### Prior art literatures

Non-Patent Literature 1: Kroemer, H. K. and Klotz, U. Clin. Pharmacokinet., vol. 23, p. 292-310, 1992
Non-Patent Literature 2: Zhang, L. et al., Eur. J. Pharm. Sci., vol. 31, p. 221-231, 2007
Non-Patent Literature 3: Renaud, S. and De Lorgeril, M., Lancet, vol. 339, p. 1523-1526, 1992
Non-Patent Literature 4: Gehm, B. D. et al., Proc. Natl. Acad. Sci. USA, vol. 94, p. 14138-14143, 1997
Non-Patent Literature 5: Corder, R. et al., Nature, vol. 414, p. 863-864, 2001
Non-Patent Literature 6: Lamming, D. W et al. Mol. Microbiol., vol. 53, p. 1003-1009,2004
Non-Patent Literature 7: Corder, R. et al., Nature, vol. 444, p. 566, 2006
Non-Patent Literature 8: Baur, J. A. and Sinclair, D. A., Nature Rev. Drug Discovery, vol. 5, p. 493-506, 2006
Non-Patent Literature 9: Baur, J. A. et al., Nature, vol. 444, p. 337-342,2006
Non-Patent Literature 10: Hmamouchi, M. et al., Am. J. Enol. Vitic., vol. 47, p. 186-192,1996
Non-Patent Literature 11: Castillo-Munoz, N. et al., J. Agric. Food. Chem., vol. 55, p. 992-1002, 2007
Non-Patent Literature 12: Juergenliemk, G. et al., Plant Med., vol. 69, p. 1013-1017, 2003
Non-Patent Literature 13: Terao, J. et al, Free Radical Res., vol. 35, p. 925-931, 2001
Non-Patent Literature 14: Shirai, M. et al., J. Agric. Food Chem., vol. 49, p. 5602-5608,2001
Non-Patent Literature 15: Shirai, M. et al., Biosci. Biotechnol. Biocham., vol. 66, p. 1015-1021,2002
Non-Patent Literature 16: Yoshimizu, M. et al., Biochem. Biophys. Res. Commu., vol. 293, p. 1458-1465, 2002
Non-Patent Literature 17: Ford, C. M. et al., J. Biol. Chem., vol. 273, p. 9224-9233, 1998
Non-Patent Literature 18: Miller, K. D. et al., J. Biol. Chem., vol. 274, p. 34011-34019, 1999
Non-Patent Literature 19: Yonekura-Sakakibara, K. et al., J. Biol. Chem., vol. 282, p. 14932-14941,2007
Non-Patent Literature 20: Yonekura-Sakakibara, K. et al., Plant Cell, vol. 20, p. 2160-2176, 2008
Non-Patent Literature 21: The French-Italian Public Consortium for Grapevine Genome Characterization, Nature, vol. 449, p. 463-468, 2007
Non-Patent Literature 22: Velasco, T. et al., PLoS ONE, vol. 12, el326, p. 1-18, 2007

### DISCLOSURE OF THE INVENTION

Therefore, the problem to be solved by the present invention is to provide an enzyme which is capable of transferring glucuronic acid to a flavonoid, especially a flavonol, at its position 3, a polynucleotide encoding the enzyme, a vector comprising the polynucleotide, a transformant obtained using the vector; and so on.

The inventors made extensive studies to solve the problem described above. As a result, the inventors focused attention on that there is the correlation between the gene sequence and structure of flavonoid UGT and its position specificity to an acceptor substrate and extracted from the genome sequence information a grapevine-derived UGT gene, which is highly homologous to the gene for enzyme capable of transferring a sugar to the position 3 of flavonoid. Thus, 6 types of candidate grapevine-derived UGT genes (Vitis vinifera UDP-sugar: glycosyltransferase; VvGT) were obtained. The inventors have found that one (VvGT5) of the 6 VvGTs is an enzyme having an activity of transferring glucuronic acid to the position 3 of flavonoid dependently on UDP-glucuronic acid, namely, a gene encoding flavonoid 3-O-glucuronosyltransferase (flavonoid 3-O-glucuronic acid transferase; F3GAT). The present invention has thus been accomplished.

That is, the present invention provides the following features.
(1) A polynucleotide of any one of (a) to (f) below:
   (a) a polynucleotide comprising a polynucleotide consisting of the nucleotide sequence represented by SEQ ID NO: 3;
   (b) a polynucleotide comprising a polynucleotide encoding a protein consisting of the amino acid sequence represented by SEQ ID NO: 4;
   (c) a polynucleotide comprising a polynucleotide encoding a protein consisting of the amino acid sequence wherein 1 to 15 amino acids are deleted, substituted, inserted and/or added in the amino acid sequence represented by SEQ ID NO: 4, and having a UDP-glucuronosyltransferase activity;
   (d) a polynucleotide comprising a polynucleotide encoding a protein that has an amino acid sequence having a homology of at least 80% to the amino acid sequence represented by SEQ ID NO: 4, and has a UDP-glucuronosyltransferase activity;
   (e) a polynucleotide comprising a polynucleotide encoding a protein that hybridizes under stringent conditions with a polynucleotide consisting of a nucleotide sequence complementary to the nucleotide sequence represented by SEQ ID NO: 3 and has a UDP-glucuronosyltransferase activity; or,
   (f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4, and has a UDP-glucuronosyltransferase activity.
      The polynucleotide of (1) described above includes, for example, a polynucleotide of any one of (g) to (j) below.
   (g) a polynucleotide comprising a polynucleotide encoding a protein consisting of the amino acid sequence wherein not greater than 10 amino acids are deleted, substituted, inserted and/or added in the amino acid sequence represented by SEQ ID NO: 4, and having a UDP-glueuronosyltransferase activity;
   (h) a polynucleotide comprising a polynucleotide encoding a protein that has an amino acid sequence having a homology of at least 90% to the amino acid sequence represented by SEQ ID NO: 4, and has a UDP-glucuronosyltransferase activity;
   (i) a polynucleotide comprising a polynucleotide encoding a protein that hybridizes under highly stringent conditions with a polynucleotide consisting of a nucleotide sequence complementary to the nucleotide sequence represented by SEQ ID NO: 3, and has a UDP-glucuronosyltransferase activity; or,
   (j) a polynucleotide comprising a polynucleotide encoding a protein that hybridizes under highly stringent conditions with a polynucleotide consisting of a nucleotide sequence complementary to the nucleotide sequence of a polynucleotide encoding a protein consisting of the amino acid sequence represented by SEQ ID NO: 4, and has a UDP-glucuronosyltransferase activity.

In the polynucleotide of (1) described above, the polynucleotides according to (c) to (j) above are, for example, the polynucleotides according to (c') to (j') below, respectively.
(c') a polynucleotide comprising a polynucleotide encoding a protein consisting of the amino acid sequence wherein 1 to 15 amino acids except for the amino acid at position 140 are deleted, substituted, inserted and/or added in the amino acid sequence represented by SEQ ID NO: 4, and having a UDP-glucuronosyltransferase activity;
(d') a polynucleotide comprising a polynucleotide encoding a protein that has an amino acid sequence having a horology of at least 80% to the amino acid sequence represented by SEQ ID NO: 4 wherein the amino acid corresponding to the amino acid at position 140 in the amino acid sequence represented by SEQ ID NO: 4 is arginine, and has a UDP-glucuronosyltransferase activity;
(e') a polynucleotide comprising a polynucleotide encoding a protein that hybridizes under stringent conditions with a polynucleotide consisting of a nucleotide sequence complementary to the nucleotide sequence represented by SEQ ID NO: 3 wherein the nucleotides corresponding to the nucleotides at positions 418-420 in the nucleotide sequence represented by SEQ ID NO: 3 are the nucleotides encoding arginine, and has a UDP-glucuronosyltransferase activity;
(f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4 wherein the nucleotides corresponding to the nucleotides at positions 418-420 in the nucleotide sequence of a polynucleotide encoding a protein consisting of the amino acid sequence represented by SEQ ID NO: 4 are the nucleotides encoding arginine, and has a UDP-glucuronosyltransferase activity;
(g') a polynucleotide comprising a polynucleotide encoding a protein consisting of the amino acid sequence wherein not greater than 10 amino acids except for the amino acid at position 140 are deleted, substituted, inserted and/or added in the amino acid sequence represented by SEQ ID NO: 4, and having a UDP-glucuronosyltransferase activity;
(h') a polynucleotide comprising a polynucleotide encoding a protein that has an amino acid sequence having a homology of at least 90% to the amino acid sequence represented by SEQ ID NO: 4 wherein the amino acid corresponding to the amino acid at position 140 in the amino acid sequence represented by SEQ ID NO: 4 is arginine, and has a UDP-glucuronosyltransferase activity;
(i') a polynucleotide comprising a polynucleotide encoding a protein that hybridizes under highly stringent conditions with a polynucleotide consisting of a nucleotide sequence complementary to the nucleotide sequence represented by SEQ ID NO: 3 wherein the nucleotides corresponding to the nucleotides at positions 418-420 in the nucleotide sequence represented by SEQ ID NO: 3 are the nucleotides encoding arginine, and has a UDP-glucuronosyltransferase activity; or,
(j') a polynucleotide comprising a polynucleotide encoding a protein that hybridizes under highly stringent conditions with a polynucleotide consisting of a nucleotide sequence complementary to the nucleotide sequence of a polynucleotide encoding a protein consisting of the amino acid sequence represented by SEQ ID NO: 4 wherein the nucleotides corresponding to the nucleotides at positions 418-420 in the nucleotide sequence of a polynucleotide encoding a protein consisting of the amino acid sequence represented by SEQ ID NO: 4 are the nucleotides encoding arginine, and has a UDP-glucuronosyltransfierase activity.

The polynucleotide of (1) described above includes, for example, a polynucleotide wherein the UDP-glucuronosyltransferase activity described above is a flavonoid 3-O-glucuronosyltransferase activity.

For example, the polynucleotide of (1) described above comprises a polynucleotide consisting of the nucleotide sequence represented by SEQ ID NO: 3, or a polynucleotide encoding a protein consisting of the amino acid sequence represented by SEQ ID NO: 4.

The polynucleotide of (1) described above is, e.g., a DNA.
(2) A protein encoded by the polynucleotide of (1) described above.
(3) A vector comprising the polynucleotide of (1) described above.
(4) A transformant, wherein the polynucleotide of (1) described above is introduced.
(5) A transformant, wherein the vector of (3) described above is introduced.
(6) A method for producing the protein of (2) described above, which comprises using the transformant of (4) or (5) above.
(7) A method for producing a glucuronate conjugate, which comprises forming the glucuronate conjugate from UDP-glucuronic acid and a glycosyl acceptor substrate using the protein (2) above as a catalyst.

The method of (7) described above includes, for example, a method in which the glycosyl acceptor substrate is a flavonoid.

### EFFECT OF THE INVENTION

In general, most flavonoids are glucuronidated *in vivo* at their position 3 or 7. It is thus expected that their functionality and usefulness will be different due to differences in structure. However, it did not reflect economic efficiency and time complexity to obtain these glucuronidated metabolites from human or non-human animals by separation and purification.

According to the present invention, glucuronic acid can be transferred to flavonoids at their position 3 by introducing the gene of the present invention in vitro or into host cells. The present invention can thus provide a sugar transferring enzyme (glycosyltransferase) which enables to readily produce useful flavonoid glucuronides including miquelianin, independently from such natural resources as described above. Furthermore, according to the present invention, there may be provided a polynucleotide encoding the enzyme, a vector comprising the polynucleotide and a transformant obtained using the vector as well as a method for producing a glucuronate conjugate using the enzyme, and so on.

### BRIEF DESCRIPTION OF DRAWINGS

FIG. 1 shows the results of phylogenetic analysis of homologous genes encoding Vitis vinifera-derived flavonoid glycosyltransferases, and is a phylogenetic tree of cluster I of flavonoid UGT genes including 8 types of Vitis vinifera UGT (VvGT) according to the Neighbor-Joining (NJ) method. The outgroup is glucosyltransferase gene (VIRSgt) of resveratrol in Vitis labrusca (Concord cultivar).
FIG. 2 shows the results of chromosomal mapping of Vitis vinifera-derived glycosyltransferase homologous genes. The VvGT1 homologous genes are located in tandem on the LG11 chromosome and the LG6 chromosome.
FIG. 3 shows the results of purification (SDS-PAGE) of Escherichia coli expression VvGT5. The band (arrow in the figure) observed in the fractions eluted with 500 mM imidazole is His-tagged fusion VvGT5 protein. "M," "Crude," "Passed" and "Washed" denote a molecular size marker, crude enzyme solution, passed-through solution and solution after washing, respectively.
FIG. 4 shows the results of enzyme activity measurements of VvGT5, specifically the HPLC charts obtained by monitoring at 360 nm. The chart at the upper row is given by the reaction solution of quercetin and VvGT5, the chart at the middle row by standard quercetin 3-glucuronide and the chart at the lower row by standard quercetin. The arrow shown in the chart at the upper row indicates the peak of the reaction product.
FIG. 5 shows the buffer system used for the optimum pH assay of VvGT5.
FIG. 6 shows the results of reaction pH dependency of VvGT5. The ordinate denotes a relative activity (%) when the highest activity was made 100%.
FIG. 7 shows the results of reaction temperature dependency of VvGT5. The ordinate denotes a relative activity (%) when the highest activity was made 100%.
FIG. 8 shows the results of glycosyl acceptor selectivity of VvGT5, wherein "n.d." denotes values lower than the detection limit.
FIG. 9 shows the results of NMR for quercetin 3-glucuronide. The bonds of the structural formula shown in bold at the lower center part are the parts that the bonds were confirmed via the HMBC (hetero-nuclear multiple-bond connectivity) measurement.
FIG. 10 shows the results of gene expression analysis of VvGT for each organ by quantitative RT-PCR. Specifically, the results are those obtained by the gene expression analysis of VvGT in Pinot Noir cultivar (left) and Cabernet Sauvignon cultivar (right). In both cultivars, the graph at the upper row shows the results of analysis for the VvGT1 gene, the graph at the middle row for the VvGT5 gene, and the graph at the lower row for the VvGT6 gene. All of the graphs indicate the relative expression level standardized by the internal control gene (VvUBQ).
FIG. 11 shows the results of LC analysis of flavonoids, wherein Q3GA denotes quercetin 3-glueuronide and Q3Glc denotes quercetin 3-glucoside.
FIG. 12 shows the results of MC analysis, wherein Q3GA denotes quercetin 3-glucuronide and Q3Glc denotes quercetin 3-glucoside.
FIG. 13 shows the results of amino acid sequence comparison (formatted alignments), wherein the location of the arginine residue (R) 140 of VvGT5 is shown in the bold frame. At3GlcT, Ph3GlcT and VvGT1 denote in this order the flavonoid 3-O-glucosyltransferases of Arabidopsis thaliana, petunia and Vitis vinifera, respectively.
FIG. 14 shows the result of glyeosyl donor selectivity of the VvGT5 mutant (VvGT5 R140W) (Conditions for enzyme reaction (Table at the upper side)). The ordinate of the graph denotes a relative activity (%) of each of wild VvGT5 (WT) and its mutant (R140W) when the highest activity of UDP-glucuronic acid and UDP-glucose was made 100%.

### BEST MODE FOR CARRYING OUT THE INVENTION

Hereinafter, the present invention is described in detail. The scope of the invention is not intended to be limited to such description. Other variations than the specific embodiments described below may also be appropriately modified and implemented without departing from the spirit of the invention. The present specification embraces JPA 2009-011065 (filed January 21, 2009) in its entirety, which is the basis for the priority of the present application. All publications cited herein, e.g., prior art literatures, documents laid open to public inspection, patent publications and other patent documents are hereby incorporated by reference into the present specification in their entirety.

### 1. Polynucleotide of the invention

First, the present invention provides (a) a polynucleotide comprising a polynucleotide consisting of the nucleotide sequence represented by SEQ ID NO: 4 (specifically a DNA, hereinafter sometimes merely referred to as "DNA"); and (b) a polynucleotide comprising a polynucleotide encoding a protein having the amino acid sequence represented by SEQ ID NO: 4. The DNA targeted in the present invention is not limited only to the DNA encoding the glucuronosyltransferase described above but also includes other DNAs encoding a protein functionally equivalent to this protein.

The functionally equivalent protein is, for example, (c) a protein consisting of an amino acid sequence with deletion, substitution, insertion and/or addition of 1 to 15 amino acids in the amino acid sequence represented by SEQ ID NO: 4, and having the UDP-glucuronosyltransferase activity. Such a protein includes, for example, a protein consisting of the amino acid sequence represented by SEQ ID NO: 4, wherein 1 to 15, 1 to 14, 1 to 13, 1 to 12, 1 to 11, 1 to 10, 1 to 9, 1 to 8, 1 to 7, 1 to 6 (1 to several), 1 to 5, 1 to 4, 1 to 3, 1 to 2 or 1 amino acid(s) is/are deleted, substituted, inserted and/or added, and having the UDP-glucuronosyltransferase activity. The number of deletions, substitutions, insertions, and/or additions of the amino acid residues described above is preferably smaller in general. In the present invention, the functionally equivalent protein of (c) described above is preferably a protein consisting of the amino acid sequence in which the amino acid corresponding to the amino acid (arginine) at position 140 in the amino acid sequence represented by SEQ ID NO: 4 is arginine as well. That is, the deletion, substitution, insertion and/or addition of the amino acid residues described above are preferably made on an amino acid(s) other than the amino acid at position 140 of the amino acid sequence represented by SEQ ID NO: 4. This preferred embodiment applies to the above polynucleotide of (g) as well.

The functionally equivalent protein includes, for example, (d) a protein that has an amino acid sequence having a homology of at least 80% to the amino acid sequence represented by SEQ ID NO: 4, and has the UDP-glucuronosyltransferase activity. Such proteins include a protein having an amino acid sequence having a homology of approximately 80% or higher, 81 % or higher, 82% or higher, 83% or higher, 84% or higher, 85% or higher, 86% or higher, 87% or higher, 88% or higher, 89% or higher, 90% or higher, 91 % or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher, to the amino acid sequence represented by SEQ ID NO: 4, and having the UDP-glucuronosyltransferase activity. As the homology percentage described above is higher, the protein is preferred in general. In the present invention, the functionally equivalent protein of (d) described above is preferably a protein consisting of the amino acid sequence, in which the amino acid corresponding to the amino acid (arginine) at position 140 in the amino acid sequence represented by SEQ ID NO: 4 is arginine as well. This preferred embodiment applies to the above polynucleotide of (h) as well.

As used herein, the term "UDP-glueuronosyltransferase activity" is intended to mean an activity of catalyzing the reaction that involves transferring glucuronic acid to the hydroxyl group in a flavonoid, which acts as a glycosyl acceptor substrate, dependently on UDP-glucuronic acid of a glycosyl donor to produce a glucuronate conjugate, namely, a flavonoid 3-O-glucuronosyltransferase (flavonoid 3-O-glucuronic acid transferase; F3GAT) activity.

The UDP-glucuronosyltransferase activity can be determined, for example, by reacting UDP-glucuronic acid with a flavonoid (e.g., a flavone, a flavonol, etc.) as a glycosyl acceptor substrate in the presence of an enzyme to be assessed and analyzing the resulting reaction product by HPLC, etc. (cf., the description in EXAMPLES later shown for more details).

Furthermore, the present invention includes (e) a polynucleotide comprising a polynucleotide encoding a protein that hybridizes under stringent conditions with a polynucleotide consisting of a nucleotide sequence complementary to the nucleotide sequence represented by SEQ ID NO: 3 and has the UDP-glucuronosyltransferase activity. In the present invention, the polynucleotide of (e) described above preferably comprises a polynucleotide, in which the nucleotides corresponding to the nucleotides at positions 418-420 (AGG) are the same "AGG" or, although not identical to, any one of "CGU," "CGC," "CGA," "CGG" and "AGA." As used herein, the nucleotides at positions 418-420 (AGG) described above are the nucleotides (codon) encoding the amino acid residue (arginine) at position 140 in the amino acid sequence represented by SEQ ID NO: 4. This preferred embodiment applies to the polynucleotide of (i) described above as well.

The present invention further includes (f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4, and has the UDP-glucuronosyltransferase activity. In the present invention, the polynucleotide of (f) described above preferably comprises a polynucleotide, in which the nucleotides corresponding to the nucleotides at positions 418-420 in the nucleotide sequence of a polynucleotide encoding a protein consisting of the amino acid sequence represented by SEQ ID NO: 4 are the same nucleotides or, although not identical to, any one of "CGU," "CGC," "CGA," "CGG," "AGA" and "AGG." As used herein, the nucleotides at positions 418-420 (AGG) described above are the nucleotides (codon) encoding the amino acid residue (arginine) at position 140 in the amino acid sequence represented by SEQ ID NO: 4. The preferred embodiment applies to the polynucleotide of (j) described above as well.

As used herein, the term "polynucleotide" refers to a DNA or RNA, preferably a DNA.

As used herein, the term "polynucleotide that hybridizes under stringent conditions" refers to, for example, a polynucleotide consisting of a nucleotide sequence complementary to the nucleotide sequence represented by SEQ ID NO: 3, or a polynucleotide obtained by colony hybridization, plaque hybridization, Southern hybridization or the like, using as a probe all or part of a polynucleotide consisting of a nucleotide sequence complementary to the nucleotide sequence of a polynucleotide encoding the amino acid sequence represented by SEQ ID NO: 4. The hybridization method which may be used includes methods described in, for example, "Sambrook & Russell, Molecular Cloning: A Laboratory Manual Vol. 3, Cold Spring Harbor, Laboratory Press 2001," "Ausubel, Current Protocols in Molecular Biology, John Wiley & Sons 1987-1997," etc.

As used herein, "stringent conditions" may refer to less stringent conditions, moderately stringent conditions and highly stringent conditions. The "less stringent conditions" are, for example, 5x SSC, 5x Denhardt's solution, 0.5% SDS, 50% formamide and 32°C. The "moderately stringent conditions" are, for example, 5x SSC, 5x Denhardt's solution, 0.5% SDS, 50% formamide and 42°C. The "highly stringent conditions," are, for example, 5x SSC, 5x Denhardt's solution, 0.5% SDS, 50% formamide and 50°C. Under these conditions, as the temperature is higher, a DNA with higher homology is expected to be obtained efficiently at higher temperature, although multiple factors are involved in the hybridization stringency including temperature, probe concentration, probe length, ionic strength, time, salt concentration and the like. Those skilled in the art may realize similar stringency by appropriately choosing these factors.

When a commercially available kit is used for hybridization, for example, Alkphos Direct Labeling Reagents (manufactured by Amersham Pharmacia) can be used. In this case, according to the protocol attached to the kit, a membrane is incubated with a labeled probe overnight, the membrane is washed with a primary wash buffer containing 0.1% (w/v) SDS at 55°C and the hybridized DNA can then be detected.

Other polynucleotides that are hybridizable include DNAs having a homology of approximately 60% or higher, approximately 70% or higher, 71 % or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91 % or higher, 92% or higher, 93% or higher, 94% or higher, 95% or higher, 96% or higher, 97% or higher. 98% or higher, 99% or higher, 99.1 % or higher, 99.2% or higher, 99.3% or higher, 99.4% or higher, 99.5% or higher, 99.6% or higher, 99.7% or higher, 99.8% or higher, or 99.9% or higher, to a DNA of the nucleotide sequence represented by SEQ ID NO: 3 or a DNA encoding the amino acid sequence represented by SEQ ID NO: 4, as calculated by homology search software, such as FASTA and BLAST using default parameters.

Homology between amino acid sequences or nucleotide sequences can be determined by using algorithm BLAST by Karlin and Altschul (Proc. Natl. Acad. Sci. USA, vol. 87, p. 2264-2268, 1990; Proc. Natl. Acad. Sci. USA, vol. 90, p. 5873, 1993). Programs called BLASTN and BLASTX based on BLAST algorithm have been developed (Altschul SF, et at., J. Mol. Biol., vol. 215, p. 403, 1990). Where a nucleotide sequence is sequenced using BLASTN, the parameters are, for example, score = 100 and word length=12. Where an amino acid sequence is sequenced using BLASTX, the parameters are, for example, score = 50 and word length = 3. Where BLAST and Gapped BLAST programs are used, default parameters for each of the programs are employed.

The polynucleotide of the invention described above can be acquired by known genetic engineering means or known synthetic means.

### 2. Protein of the invention

In a further embodiment, the present invention also provides the protein encoded by the polynucleotides of the present invention described above. The protein of the invention in an embodiment is a protein consisting of the amino acid sequence represented by SEQ ID NO: 4. The protein of the invention in another embodiment is a protein having the amino acid sequence represented by SEQ ID NO: 4.

The protein of the present invention in a further embodiment is a protein consisting of an amino acid sequence with deletion, substitution, insertion and/or addition of 1 to 15 amino acids in the amino acid sequence represented by SEQ ID NO: 4, and having the UDP-glucuronosyltransferase activity. Such a protein includes a protein having an amino acid sequence with the homology as described for the amino acid sequence represented by SEQ ID NO: 4 and having the UDP-glucuronosyltransferase activity. These proteins may be obtained by using site-directed mutagenesis described in "Sambrook & Russell, Molecular Cloning: A Laboratory Manual Vol. 3, Cold Spring Harbor, Laboratory Press 2001," "Ausubel, Current Protocols in Molecular Biology, John Wiley & Sons 1987-1997," "Nuc. Acids. Res., vol. 10, p. 6487, 1982," "Proc. Natl. Acad. Sci. USA, vol. 79, p. 6409, 1982," "Gene, vol. 34, p. 315, 1985," "Nuc. Acids. Res., vol. 13, p. 4431, 1985," "Proc. Natl. Acad. Sci. USA, vol. 82, p. 488, 1985," etc. In a still further embodiment, the protein described above is preferably a protein consisting of the amino acid sequence in which the amino acid residue corresponding to the amino acid residue (arginine) at position 140 in the amino acid sequence represented by SEQ ID NO: 4 is arginine as well. That is, the deletion, substitution, insertion and/or addition of the amino acid residues described above are preferably made on an amino acid(s) other than the amino acid at position 140 of the amino acid sequence represented by SEQ ID NO: 4.

The deletion, substitution, insertion and/or addition of one or more (e.g., 1 to 15, preferably 10 or less) amino acid residues in an amino acid sequence of the protein of the present invention means that one or a plurality of amino acid residues are deleted, substituted, inserted and/or added at one or a plurality of positions in the same amino acid sequence. Two or more types of deletion, substitution, insertion and addition may occur concurrently.

Examples of amino acid residues which are mutually substitutable are given below. Amino acid residues in the same group are mutually substitutable. Group A: leucine, isoleucine, norleucine, valine, norvaline, alanine, 2-aminobutanoic acid, methionine, o-methylserine, t-butylglycine, t-butylalanine and cyclohexylalanine; Group B: aspartic acid, glutamic acid, isoaspartic acid, isoglutamic acid, 2-aminoadipic acid and 2-aminosuberic acid; Group C: asparagine and glutamine; Group D: lysine, arginine, ornithine, 2,4-diaminobutanoic acid and 2,3-diaminopropionic acid; Group E: proline, 3-hydroxyproline and 4-hydroxyproline; Group F: serine, threonine and homoserine; and Group G: phenylalanine and tyrosine.

The protein of the present invention may also be produced by chemical synthesis methods such as the Fmoc method (fluorenylmethyloxycarbonyl method) and the tBoc method (t-butyloxycarbonyl method). In addition, peptide synthesizers available from Advanced ChemTech, Perkin Elmer, Pharmacia, Protein Technology Instrument, Synthecell-Vega, PerSeptive, Shimadzu Corp., etc. may also be used for the chemical synthesis.

Herein, the protein of the invention is a flavonoid 3-O-glucuronosyltransferase. The term "glucuronosyitransferase" catalyzes the reaction of transferring the glucuronyl group from a glycosyl donor onto a glycosyl acceptor substrate to form the glucuronate conjugate. In the present invention, the glycosyl acceptor substrate is a flavonoid and the glycosyl donor is UDP-glucuronic acid. In an embodiment of the present invention, the protein catalyzes the reaction of transferring the glucuronic acid residue from UDP-glucuronic acid to the hydroxy group at position 3 of a flavonoid (e.g., a flavonol, a flavone) as the glycosyl acceptor substrate to form the glucuronate conjugate and UDP.

The flavonoid as the glycosyl acceptor substrate includes flavones, flavonols, flavanones, isoflavones, flavone C-glycosides, aurones, catechins, and the like. Among them, examples of the flavones include baicalein, scutellarein, apigenin, luteolin, tricetin, diosmetin, chrysoeriol, etc.; examples of the flavonols include quercetin, myricetin, larycitrin, isorhamnetin, syringetin, kaempferol, etc. An example of the flavanones is naringenin. Examples of the isoflavones are genistein, daidzein and formononetin. Examples of the flavone C-glycosides include vitexin, isovitexin and orientin. An example of the aurones is aureusidin. Examples of the catechins are catechin and epigallocatechin gallate. In the present invention, flavones and flavonols are, among others, preferred as the glycosyl acceptor substrate flavonoids, more preferably flavones and flavonols, and most preferably quercetin, kaempferol and myricetin.

### 3. Vector and transformant bearing the same

In another embodiment, the present invention provides the expression vector comprising the polynucleotide of the present invention. The expression vector of the invention comprises the polynucleotide of the present invention (any one of the polynucleotides (a) to (j) described above). Preferably, the expression vector of the invention comprises any one of the polynucleotides (g) to (j) described above. More preferably, the expression vector of the invention comprises a polynucleotide consisting of the nucleotide sequence of SEQ ID NO: 3, or a polynucleotide comprising a polynucleotide encoding a protein consisting of the amino acid sequence represented by SEQ ID NO: 4.

The vector of the invention is generally constructed to contain an expression cassette comprising as constituent elements (i) a promoter that is transcribable in a host cell, (ii) the polynucleotide of the present invention (e.g., any one of the polynucleotides described in (a) to (j) above) that is linked to the promoter, and (iii) a signal that functions in the host cell with respect to the transcription termination and polyadenylation of RNA molecule. The vector thus constructed is introduced into a host cell. To construct the expression vector, methods using a plasmid, phage or cosmid are used but are not particularly limited thereto.

Specific types of the vector are not particularly limited, and vectors capable of expressing in a host cell can be suitably chosen. That is, a suitable promoter sequence may be chosen depending upon the type of a host cell to reliably express the polynucleotide of the invention, and a vector obtained by incorporating this sequence and the polynucleotide of the present invention into various plasmids or the like may be used as an expression vector.

The expression vector of the present invention contains an expression control region (for example, a promoter, a terminator, and/or a replication origin, etc.) depending on the type of a host to be introduced. A conventional promoter (for example, trc promoter, tac promoter, lac promoter, etc.) is used as the promoter for a bacterial expression vector. As the promoter for yeast, there are used, for example, a glyceraldehyde 3-phosphate dehydrogenase promoter, PH05 promoter, etc. As the promoter for fungi there are used, for example, amylase, trpC, etc. Additionally, a viral promoter (e.g., SV40 early promoter, SV40 late promoter, etc.) is used as the promoter for animal-derived host cell.

The expression vector preferably contains at least one selective marker. The marker available includes an auxotrophic marker (ura5, niaD), a drug-resistant marker (hygromycin, zeocin, geneticin), a copper-resistant gene (CUP1) (Marin et al., Proc. Natl. Acad. Sci. USA, vol. 81, p. 337, 1984), a cerulenin-resistant gene (fas2miri, PDR4) (Junji Inokoshi et al., Biochemistry, 6 vol. 64, p. 660, 1992; Hussain et al., Gene, vol. 101, p. 149, 1991, respectively), and the like.

The present invention also provides the transformant in which the polynucleotide of the present invention (e.g., the polynucleotide described in any of (a) to (j) above) is introduced.

A method of preparing (method of producing) the transformant is not particularly limited but includes, for example, a method which comprises introducing the recombinant vector into a host followed by transformation. The host cell as used herein is not particularly limited and various known cells may be preferably used. Specific examples are bacteria such as Escherichia coli, etc., yeast (budding yeast Saccharomyces cerevisiae, fission yeast Schizosaccharomyces pombe), nematode (Caenorhabditis elegans), oocyte of African clawed frog (Xenopus laevis), etc. Culture media and conditions suitable for the host cells above are well known in the art. The organism to be transformed is not particularly limited, and includes various microorganisms, plants and animals given as examples of the host cells above.

For transformation of the host cell, there may be used generally known methods. For example, methods used include but not limited to the electroporation method (Mackenxie D. A. et al., Appl. Environ. Microbiol., vol. 66, p. 4655-4661, 2000), the particle delivery method (the method described in JPA 2005-287403 "Method of Breeding Lipid-Producing Fungus"), the spheroplast method (Proc. Natl. Acad. Sci. USA, vol. 75, p. 1929, 1978), the lithium acetate method (the method described in J. Bacteriology, vol. 153, p. 163, 1983), and the methods described in Methods in yeast genetics, 2000 Edition: A Cold Spring Harbor Laboratory Course Manual, etc.

In another embodiment of the present invention, the transformant can be a plant transformant. The plant transformant in accordance with this embodiment can be acquired by introducing a recombinant vector bearing the polynucleotide in accordance with the present invention into a plant in such a manner that the polypeptide encoded by said polynucleotide can be expressed.

Where a recombinant expression vector is used, the recombinant expression vector used to transform the plant is not particularly limited as far as the vector is capable of expressing the polynucleotide in accordance with the present invention in said plant. Examples of such vectors include a vector bearing a promoter capable of constitutively expressing the polynucleotide in plant cells (e.g., a 35S promoter of cauliflower mosaic virus) in plant cells, and a vector inducibly activated by external stimulation.

Plants which are to be the target of transformation in the present invention may be any of entire plant bodies, plant organs (e.g., leaves, petals, stems, roots, seeds, etc.), plant tissues (e.g., epidermis, phloem, parenchyma, xylem, vascular bundles, palisade tissues, spongy tissues, etc.) or plant culture cells, or various types of plant cells (e.g., suspension culture cells), protoplasts, leaf slices, callus, and the like. Plant species used for transformation are not particularly limited and include any of plants belonging to the Monocotyledoneae and the Dicotyledoneae.

For transformation of genes into plants, conventional transformation methods known to those skilled in the art (e.g., the Agrobacterium method, gene gun, the PEG method, the electroporation method, etc.) are used. For example, the Agrobacterium-mediated method and the method of directly introducing into plant cells are well known. When the Agrobacterium method is used, the constructed plant expression vector is introduced into an appropriate Agrobacterium strain (e.g., Agrobacterium tumefaciens), followed by infection of aseptically cultured leaf discs with this strain according to the leaf disc method (Hirobumi Uchimiya, Manuals for Plant Gene Manipulation (1990), 27-31, Kodansha Scientific Co., Ltd., Tokyo), whereby the transgenic plant can be obtained. The method of Nagel, et al. (Micribiol. Lett., 67, 325 (1990)) may also be used. This method involves first introducing, e.g., an expression vector into Agrobacterium and then introducing the transformed Agrobacterium into plant cells or plant tissues according to the method described in Plant Molecular Biology Manual (S. B. Gelvin, et. al., Academic Press Publishers). As used herein, the "plant tissue" includes callus, which is obtained by culturing plant cells. When the transformation is carried out using the Agrobacterium method, binary vectors (pBI121 or pPZP202, etc.) can be used.

For direct transfer of genes into plant cells or plant tissues, the electroporation method and the gene gun method are known. When the gene gun is used, entire plant bodies, plant organs or plant tissues per se may be used, or may be used after preparation of protoplasts. The samples thus prepared can be bombarded using a gene transfer apparatus (e.g., PDS-1000 (BIO-RAD, Inc.), etc.). Bombardment conditions vary depending upon type of the plant or sample. Normally, the sample is bombarded under a pressure of about 450-2000 psi at a distance of 4-12 2 cm.

The cells or plant tissues in which the gene is introduced are first selected by chemical resistance such as hygromycin resistance, etc. and then regenerated into plant bodies in a conventional manner. Regeneration of plant bodies from the transformant cells can be performed by methods known to those skilled in the art, depending upon kind of plant cells.

Where a plant culture cell is used as a host, transformation is preformed by introducing the recombinant vector into culture cells by the gene gun method, the electroporation method, etc. Callus, shoots, hairy roots, etc. resulted from the transformation can be used directly in cell culture, tissue culture or organ culture. Furthermore, they can be regenerated into plant bodies by conventional plant tissue culture methods through administration of plant hormones (e.g., auxin, cytokinin, gibberellin, abscisic acid, ethylene, brassinolide, etc.) at appropriate concentrations.

Whether or not the gene is introduced into the host can be confirmed by PCR, Southern hybridization, northern hybridization, or the like. For example, a DNA is prepared from the transgenic plant and DNA-specific primers are then designed for PCR. PCR can be performed under the same conditions as used for the preparation of plasmids described above. Subsequently, the amplified product is subjected to agarose gel electrophoresis, polyacrylamide gel electrophoresis, capillary electrophoresis, etc. and stained with ethidium bromide, SYBR Green solution, etc. By detecting the amplified product as a single band, it can be confirmed that transformation has occurred. Alternatively, PCR may be performed using primers previously labeled with a fluorescent dye or the like, and the amplified product can then be detected. In addition, there may be employed such a method that the amplified product is bound to the solid phase of a microplate or the like to confirm the amplified product by means of fluorescence or enzyme reactions, or the like.

Once the transgenic plant wherein the polynucleotide in accordance with the present invention has been integrated into the genome is acquired, its progeny can be obtained by sexual or asexual reproduction of the plant body. Also, the plant body can be mass-produced by acquiring from the plant body or its progeny or clones thereof, e.g., seeds, fruits, cut panicles, tubers, tuberous roots, strains, callus, protoplasts, etc., and then using them as the origin. Accordingly, the present invention further encompasses the plant body in which the polynucleotide in accordance with the present invention is expressibly introduced, or progenies of the plant body having the same property as in the plant body, and tissues derived therefrom.

Moreover, the transformation methods for various plants are already reported. Examples of the transgenic plants in accordance with the present invention include, but not be limited to, grape, sesame, rice plant, tobacco, barley, wheat, rapeseed, potato, tomato, poplar, banana, eucalyptus, sweet potato, soybean, alfalfa, lupinus, corn, cauliflower, rose, chrysanthemum, carnation, snapdragon, cyclamen, orchid, Prairie gentian, freesia, gerbera, gladiolus, gypsophila, kalancoe, lily, pelargonium, geranium, petunia, torenia, tulip, Forsythia intermedia, Arabidopsis thaliana, Lotus japonicus, and so on.

In an embodiment of the present invention, the transgenic plant is a plant for functional food materials.

### 4. Method for producing the protein of the invention

In yet another embodiment, the present invention provides a method for producing the protein of the present invention using the transformants described above.

Specifically, the protein of the invention may be obtained by isolating and purifying the protein of the invention from the culture of the transformant described above. As used herein, the culture refers to any of a culture broth, cultured bacteria or cultured cells, and the homogenate of cultured bacteria or cultured cells. Conventional methods may be used to isolate and purify the protein of the invention.

Specifically, when the protein of the invention accumulates within cultured bacteria or within cultured cells, a crude extract of the protein of the invention may be obtained by culturing the bacteria or cells, then disrupting the bacterial or cells using a conventional technique (e.g., ultrasonication, lysozymes, freezing and thawing, etc.) and applying a conventional method (e.g., centrifugation, filtration, etc.) When the protein of the invention is accumulated in the culture broth, the culture supernatant containing the protein of the invention can be obtained, after completion of the incubation, by separating the bacteria or cells from the culture supernatant in a conventional manner (e.g., centrifugation, filtration, etc.).

The protein of the invention contained in the extract or culture supernatant obtained as described above can be purified in accordance with a conventional method of separation and purification. The separation and purification including, e.g., ammonium sulfate precipitation, gel filtration chromatography, ion exchange chromatography, affinity chromatography, reversed phase high performance liquid chromatography, dialysis, and ultrafiltration, etc. may be used singly or in a suitable combination.

### 5. Method for producing the glucuronate conjugate

The present invention further provides a method for producing the glucuronate conjugate using the protein of the present invention. The protein of the invention catalyzes the reaction of transferring glucuronic acid from UDP-glucuronic acid as the glycosyl donor to a flavonoid as the glycosyl acceptor substrate (more specifically, transferring to the hydroxy group at position 3 of a flavonoid). Therefore, the glucuronate conjugate can be produced from the glycosyl acceptor substrate and the glycosyl donor by using the protein of the invention. Among the flavonoid, the glycosyl acceptor substrate is preferably a flavone and a flavonol, and most preferably quercetin, kaempferol and myricetin.

The glucuronate conjugate can be produced, for example, by preparing a solution containing 1 mM glycosyl acceptor substrate, 2 mM glycosyl donor, 50 mM calcium phosphate buffer (pH 7.5) and 20 µm of the protein of the invention and reacting them at 30°C for 30 minutes. The glucuronate conjugate can be isolated and purified from this solution by known methods. Specifically, e.g., ammonium sulfate precipitation, gel filtration chromatography, ion exchange chromatography, affinity chromatography, reversed phase high performance liquid chromatography, dialysis, ultrafiltration, etc. can be used alone or in an appropriate combination.

The glucuronate conjugate thus obtained is useful as a material for functional food, a reagent for inspecting the in vivo functions, an antioxidant, etc. (Gao, Z., Huang, K., Yang, X., and Xu, H., Biochimica et Biophysica Acta, vol. 1472, p. 643-650, 1999).

Hereinafter, the present invention is described in more details with reference to EXAMPLES below but is not deemed to be limited thereto.

### EXAMPLE 1

### Gene Cloning

In the following EXAMPLES, the molecular biological techniques described in Molecular Cloning (Sambrook et al., Cold Spring Harbour Laboratory Press, 2001) were used unless otherwise indicated in detail.

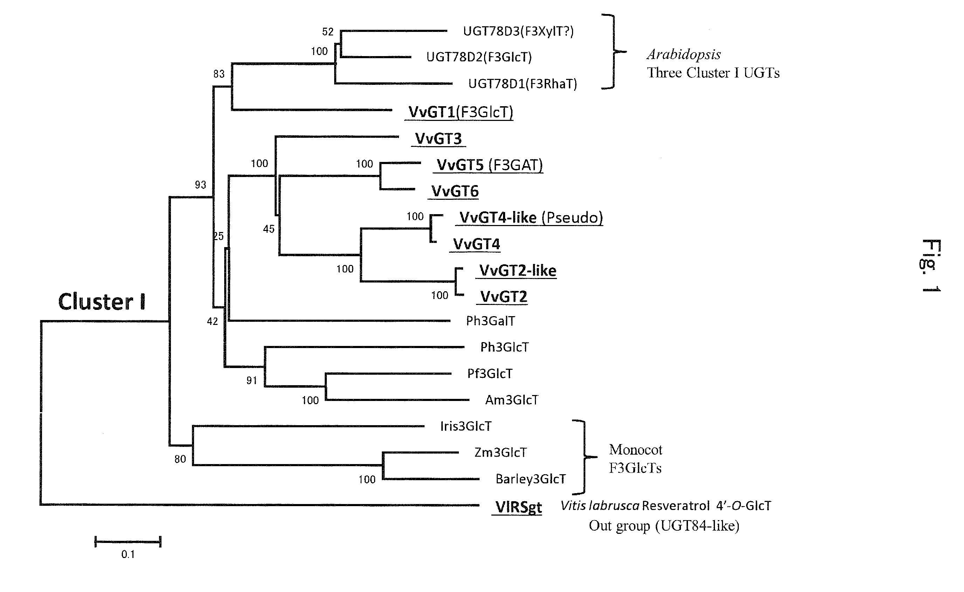

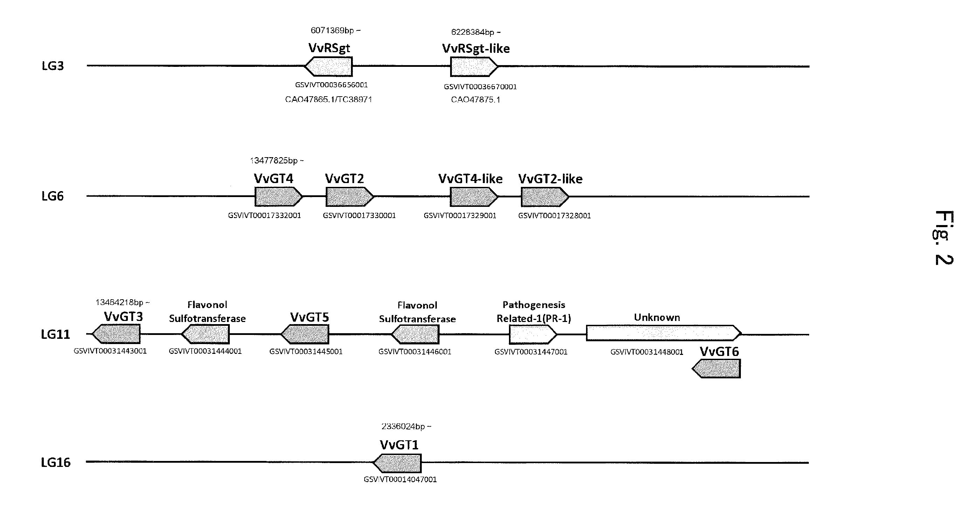

Based on the full-length sequence of Vitis vinifera-derived flavonoid 3-O-glucosyltransferase gene VvGT1 (Offen, W. et al., EMBO J., vol. 25, p. 1396-1405, 2006), a Blast homology search was performed on the Vitis vinifera genome database (http://genomics.research.iasma.it/iasma/) provided by Istituto Agrario San Michele all'Adige (LASMA). As a result, 7 candidate UGT genes showing high homology were found (FIG. 1). It was confirmed by the NJ phylogenetic analysis using the MEGA4 program (Tamura, K. et al., Mol. Biol. Evol., vol. 24, p. 1596-1599, 2007) that they belong to Cluster 1, which is a cluster of flavonoid 3-UGT (FIG. 1). Furthermore, the candidate gene sequence was compared with the physical map of chromosome to clarify synteny on the chromosome (FIG. 2). VvGT3, VvGT5 and VvGT6 are located on chromosome 11 in the same direction of transcription, and flavonol sulfotransferase-like genes were found in common between them (Varin, L. et al., Proc. Natl. Acad. Sci. USA, vol. 89, p. 1286-1290, 1992). In particular, VvGT5 and VvGT6 had high homology and were considered paralogs generated by gene duplication. Furthermore, VvGT2 and VvGT4 located in the same direction of transcription were found on chromosome 6. Copies of the set of VvGT2 and VvGT4 were found at the downstream in the same transcriptional direction and named in turn VvGT2-like and VvGT4-like, respectively. On the other hand, known VVGT1 was located on chromosome 16.

For cloning of these VvGT genes, grapevine leaf-derived cDNA was then prepared by the following procedures. Using the cloned genes as template, amplification was performed by PCR. Using FruitMate for RNA Purification and Fast Pure RNA Kit (TaKaRa Bio K.K.), the total RNA was extracted from 0.1 g of grapevine (Vitis vinifera, cultivar: Cabernet Sauvignon) leaves by the procedures recommended by the manufacturer, and cDNA was synthesized from 1 µg of the total RNA using SuperScript First-Strand Synthesis System for RT-PCR (Invitrogen, Inc.) by the procedures recommended by the manufacturer.

Taking the VvGT5 gene as an example, the procedures ofcDNA cloning and expression vector construction are given below. Using as a template the cDNA obtained by reverse transcription, it was attempted to isolate VvGT5 by PCR using the following VvGT5 gene-specific primers (SEQ ID NOS: 1 and 2) designed based on genome sequence information (GenBank Accession Number: CAN74919).

Specifically, the PCR solution (50 µl) as composed of 1 µl of grapevine leaf-derived cDNA, 1 x ExTaq buffer (TaKaRaBio), 0.2 mM dNTPs, 0.4 pmol each/µl of the primers (SEQ ID NOS: 1 and 2) and 2.5 units of ExTaq polymerase. PCR was performed, after reacting at 94°C for 3 minutes, with total 35 amplification cycles, the reaction of one cycle consisting of 94°C for 1 minute, 50°C for 1 minute and 72°C for 2 minutes.
CACC-Ndel-VvGT5-Fw:
   5'- CAC CCA TAT GAC TAC CAC CGC CAG CTC CAT -3' (SEO ID NO: 1)
BglII-VvGT5-RV:
   5'- AGA TCT CTA CTT ATT GGT GTC CAA AGG TA -3' (SEQ ID NO: 2)

The PCR solution was separated by 0.8% agarose gel electrophoresis to give the amplified fragment of about 1.4 kb size. The amplified fragment was subcloned to pENTR-Directional-TOPO vector (Invitrogen, Inc.). The nucleotide sequence of the inserted fragment was determined on DNA Sequencer Model 3100 (Applied Biosystems) by the primer walking method using synthetic oligonucleotide primers. It was confirmed that the inserted cDNA was VvGT5 (SEQ ID NOS: 3 and 4). This sequence showed 99% sequence homology to the Pinot Noir cultivar-derived sequence registered on the database (GenBank Accession Number: CAN74919) on an amino acid sequence level. This difference was considered to be due to polymorphism between grapevine cultivars.

cDNA Sequence of VvGT5:
ATGACTACCACCGCCAGCTCCATGGACAGGCATGTTGCAGTATTAGGGTTC
CCCACCCATACAGCTACCCTCTTAAAACTCCTGCGCAGACTAGCATCTGCC
GCACCCACCACCATCTTCTCCTTCTTCAACACTCCCAAGGCCAACAGCTCC
ATCTCCTCTGCTCAAAGTCCCCACGGTATTCACAATCTAAGAGTTTATGATG
TAGCAGACGGCGTGCCAGAGGACCTTGTGCTTTCAGCAAACCCCCTGGCA
CGCATCGAGATGTTCTTGAAGGCGACGCCTGGGAACTTTCGTGATGCTTTG
GAAGTGGCTGAGAAGGACATTGGAAGGAAGATAAGTTGCCTGGTGAGTG
ATGTCTTTTTGTGGTTCACTGCTGATATGGCTGAGGAAATGGGGGTTCCGT
GGGTGGCAATTAGGACTGCTGCGCTTTACTCACTCTCCGTCCACATTTATA
CTGATGCTATCCGGGAAGCAGTGGGAGTTGCGGGCAGGTGCAAGACCA
AACCCTTGATTTCATCCCAGGATTCTCAGCAATAAAGGTTGAAGACCTACC
TGAAGGAATGGTTTTCGGGGACACAGAATCTCCCTTCGCATGCATGTTGCA
TAAAATGGGGCTCATGCTGCCACGAGCAACCATTGTTGCCACAAATTCCTT
TGAAGAACTAGAGCCGACCATTGTCACAAATGATCTCAAGTCCAAGCTCC
AAAAAGTTCTTACTGTTGGTCCTTTTGACCTATCTTCACCACCACCGCTGA
TATTGGACGCCAGTGGCTGCCTGCCATGGTTGGACAATAAAAAGAAGCG
TCAGTGGCATATGTTAGTTTTGGAAGCATAGCAACACCACCACCCAACGA
GATTGTAGCATTGGCAGAAGCCCTAGAAGCAACTGGGATACCGTTTCTTTG
GTCTCTTAGGGAGCATGCAATGAACAATTTACCAAAAGGATTTCTAGAGA
GGACGACCGCTCATGGAAAAGTAGTTTCGTGGGCACCCCAGCCTCAGGTC
TTAGCACATGCCTCAGTTGCAGTGTTTATTACTCATAGCGGTTGGAACTCG
GTGACTGAGAGTATAGTTGGTGGTGTGCCCATGATCTGCAGGCCATTCTTT
GGAGATCAACGTCTTAACAGGCGGATGGTACAGGATGTATGGGGGATTGG
TATAGGAGTTGAGGGAGGGATCCTCACAAAAAGGGGAGTAATGAGTGCTT

TGGGACTAATTTTGTCCCATGAAGGGAAGAAAATGAGAGAGAAAATTGGG
GTCCTAAAAGAGCTTGCTAGAAGGGCTGTTGAACCAAACGGGAGCTCAA
CTCAAAATTTAGGTCATTTGTTGGAGGTAATCACAACATCTAAGTTACCTTT
GGACACCAATAAGTAG (SEQ ID NO: 3)

Amino acid sequence of VvGT5:
MTTTASSMDRHVAVLGFPTHTATLLKLLRRLASAAPTTIFSFFNTPKANSSISS
AQSPHGIHNLRVYDVADGVPEDLVLSANPLARIEMFLKATPGNFRDALEVAE
KDIGRKISCLVSDVFLWFTADMAEEMGVPWVAIRTAALYSLSVHIYTDAIREA
VGVAGQVQDQTLDFIPGFSAIKVEDLPEGMVFGDTESPFACMLHKMGLMLP
RATIVATNSFEELEPTIVTNDLKSKLQKVLTVGPFDLSSPPPLILDASGCLPWLD
NKKEASVAYVSFGSIATPPPNEIVALAEALEATGIPFLWSLREHAMNNLPKGFL
ERTTAHGKVVSWAPQPQVLAHASVAVFITHSGWNSVTESIVGGVPMICRPFF
GDQRLNRRMVQDVWGIGIGVEGGILTKRGVMSALGLILSHEGKKMREKIGV
LKELARRAVEPNGSSTQNLGHLLEVITTSKLPLDTNK (SEQ ID NO: 4)

Utilizing the restriction enzyme sites ofNdeI and BglII in the primers, the VvGT5 fragment of about 1.4 kb was excised and ligated to the Ndel and BamHI sites of Escherichia coli expression vector pET15b to give the Escherichia coli expression vector of VvGT5. In constructing the Escherichia coli expression vector, His tag located upstream the NdeI site of the vector was fused with the open reading frame of VvGT5 so that the vector was so designed as to express the chimeric VvGT5 protein fused with His tag.

### EXAMPLE 2

### Function Analysis of Enzyme

In order to elucidate the biochemical functions of the enzyme, the enzyme was expressed in Escherichia coli.

Using the plasmid obtained above, Escherichia coli BL21 (DE3) was transformed in a conventional manner. The resulting transformant was shake cultured overnight at 37°C in 4 ml of LB medium (10 g/l tryptone peptone, 5 g/l yeast extract, 1 g/l NaCl) supplemented with 50 µg/ml of ampicilline. The culture broth, 4 ml, which reached the stationary phase, was inoculated into 80 ml of the medium with the same composition, followed by shake culture at 37°C. When the cell turbidity (OD600) reached approximately 0.5, 0.5 mM IPTG in a final concentration was added to the medium, followed by shake culture at 18°C for 20 hours.

All of the subsequent procedures were performed at 4°C. The transformants cultured were centrifuged (5,000 x g, 10 mints.) to collect the cells, and 1 ml/g cell of Buffer S [20 mM HEPES buffer (pH 7.5), 20 mM imidazole, 14 mM β-mercaptoethanol] was added to the cells for suspension. Subsequently, ultrasonication was performed (15 secs. x 8) followed by centrifugation (15,000 x g, 15 mins.). The supernatant obtained was recovered as a crude enzyme solution. The crude enzyme solution was loaded onto His SpinTrap (GE Healthcare), which had been equilibrated with Buffer S, and centrifuged (70 x g, 30 secs.) to purify the His-tagged VvGT5 expression protein. After washing with the buffer, the protein bound to the column was stepwise eluted with 5 ml each of Buffer S containing 100 and 500 mM imidazole. The buffer in each fraction eluted was replaced by 20 mM HEPES buffer (pH 7.5) and 14 mM β-mercaptoethanol, using Microcon YM-30 (Amicon) (magnification of dialysis, x 1000).

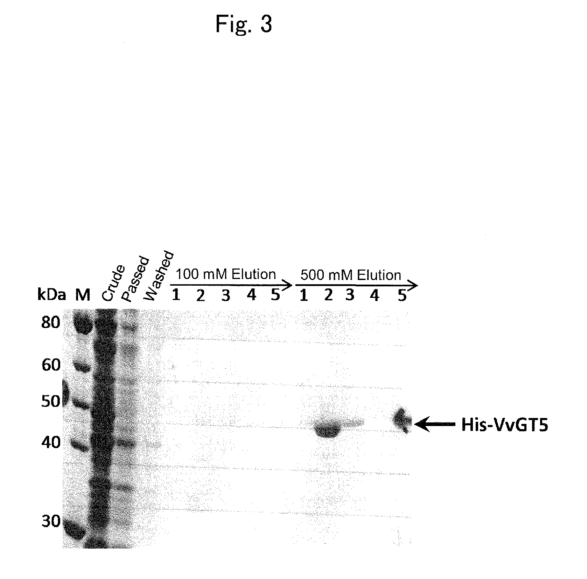

As a result of the SDS-PAGE analysis, a protein around 49.68 kDa for the estimated molecular weight of VvGT5 was detected in the fraction eluted with 500 mM imidazole. This fraction was used for the enzyme analysis (FIG. 3).

Standard conditions for the enzyme reaction were as follows. After 50 µl of a reaction solution (1 mM glycosyl donor, 200 µm glycosyl acceptor substrate, 20 mM HEPES buffer (pH 7.5), about 2 µg of purified enzyme) was prepared, the enzyme solution was added thereto to initiate the reaction. The reaction was carried out at 30°C overnight. The reaction was stopped by adding 5 µl of 20% phosphoric acid, followed by reversed phase HPLC analysis.

The reversed phase HPLC system consists of the following devices. MODEL 305 (Gilson Inc.) was used as Pump A, MODEL 302 (Gilson Inc.) as Pump B, MODEL 231 (Gilson Inc.) as Sample Injector, 401 (Gilson Inc.) as Dilutor, 811B (Gilson Inc.) as DYNAMIC MIXER, Pressure Module (RAININ), DGU-12A (Shimadzu Corporation) as DEGASSER and SPD-M20A DIODE ARRAY DETECTOR (Shimadzu Corporation) as a detector.

Conditions for HPLC of flavonols were as follows. J'sphere ODS-M80 (4.6 x 150 mm, YMC) was used as a column at room temperature and moving phase A (0.2% formic acid/10% CH₃CN) and moving phase B (0.2% formic acrid/90% CH₃CN) were used. Conditions for elution were as follows: Equilibration was performed with B 10% for 3 minutes, followed by linear density gradients (B10%→ B40%) for 10 minutes and (B40%→B90%) for further 10 minutes, which was maintained for 1 minute with B90%. Thereafter, the condition was again reverted to B10% to equilibrate for 10 minutes. The flow rate was 0.7 ml/min. Detection was performed at 360 nm using PD-M20A DIODE ARRAY DETECTOR. Under the conditions, standard quercetin, quercetin 3-glucuronide, kaempferol and rhamnetin were eluted in this order at retention times of approximately 17.8 minutes, 11.8 minutes, 20.1 minutes and 20.4 minutes, respectively Quercetin 3-glucuronide used was prepared from grapevine leaves by the method of REFERENCE EXAMPLE 1 later described.

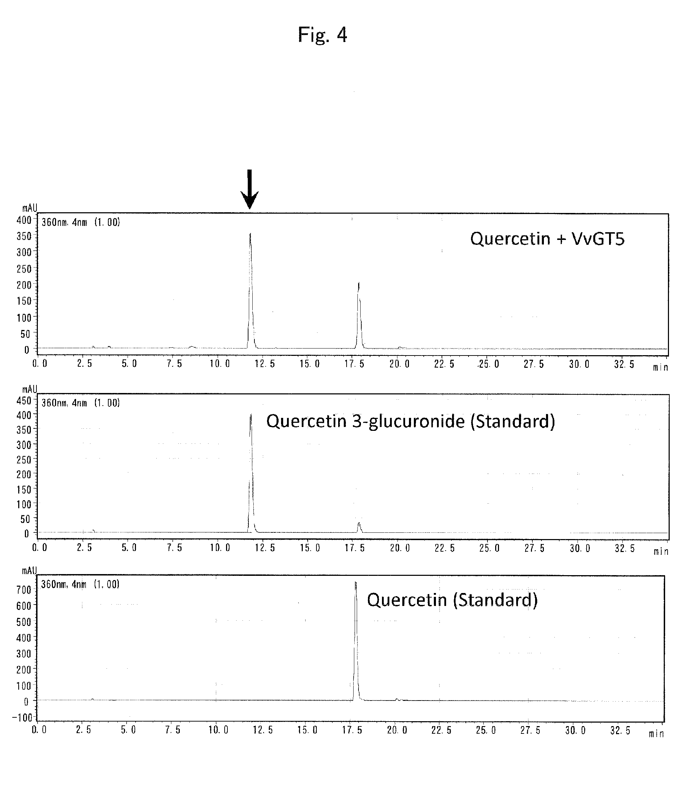

As a result of the HPLC analysis of the enzyme reaction solution using quercetin as the glycosyl acceptor and UDP-glucuronic acid as the glycosyl donor, a new product was confirmed with the retention time of about 11.8 minutes which coincided with quercetin 3-glucuronide (FIG. 4). The results reveal that the VvGT5 gene encoded F3GAT having the activity of transferring glucuronic acid to the 3 position of the flavonoid.

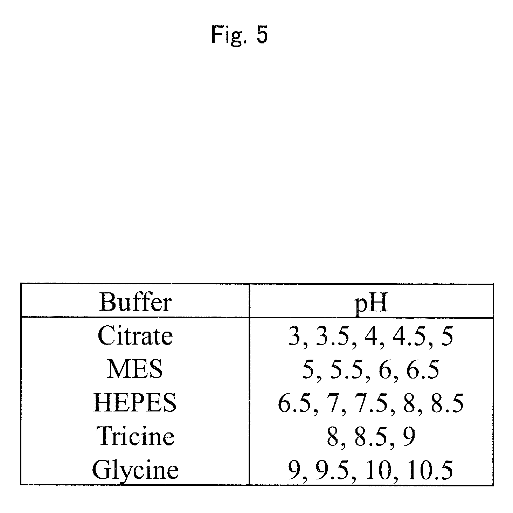

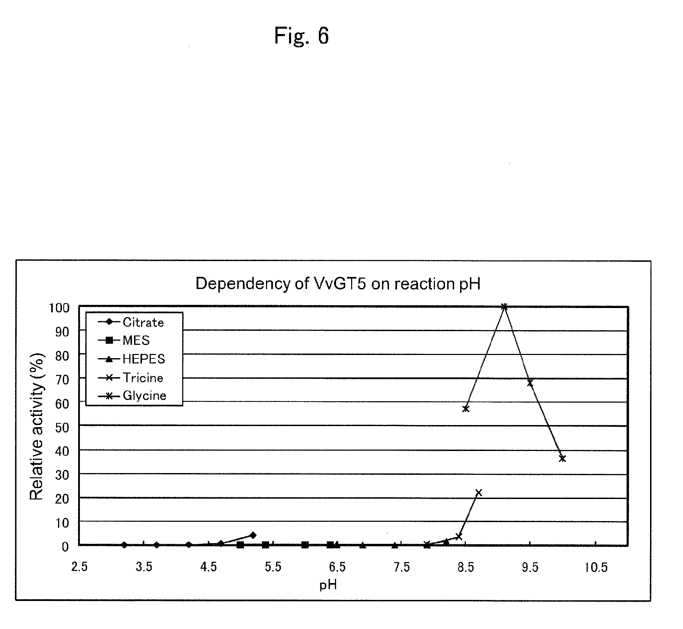

Next, the optimum pH of this enzyme was determined. As shown in FIG. 5, various buffer solutions ranging from pH 3 to pH 10.5 were prepared, and 100 µm quercetin as a glycosyl acceptor, 1 mM UDP-glucuronic acid as a glycosyl donor, 50 mM buffer, 14 mM 2-mercaptoethanol and 50 ng of the purified VvGT5 described above were reacted at 30°C for 5 minutes. The reaction was started by adding quercetin. The reaction was stopped by adding an equal volume of 40% acetonitrile solution containing 0.1% TFA. After the reaction was stopped, the solution was centrifuged (15000 rpm, 2 mins., 4°C) and 100 µl of the resulting supernatant was provided for the HPLC analysis. The amounts of the products produced under various buffer conditions were compared. As a result, a potent enzyme activity was observed in the neutral to basic region and the optimum pH was 9.1 (FIG. 6).

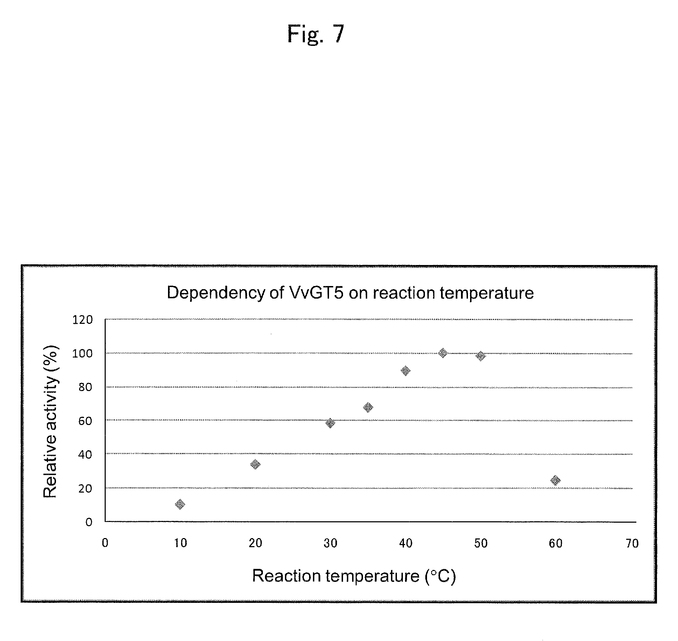

Next, the optimum pH of this enzyme was determined. As a glycosyl acceptor 100 µM quercetin, 1 mM UDP-glucuronic acid as a glycosyl donor, 50 mM glycine-NaOH (pH 9.5) buffer, 14 mM 2-mercaptoethanol and 50 ng of the purified VvGT5 described above were reacted at 10, 20, 30, 35, 40, 45, 50 and 60°C, respectively, for 15 minutes. The reaction was started by adding quercetin. The reaction was stopped by adding an equal volume of 40% acetonitrile solution containing 0.1% TFA. After the reaction was stopped, the solution was centrifuged (15000 rpm, 2 mins., 4°C) and 100 µl of the resulting supernatant was provided for the HPLC analysis. The amounts of the products produced under various temperature conditions were compared. As a result, the optimum reaction temperature was 45°C (FIG. 7).

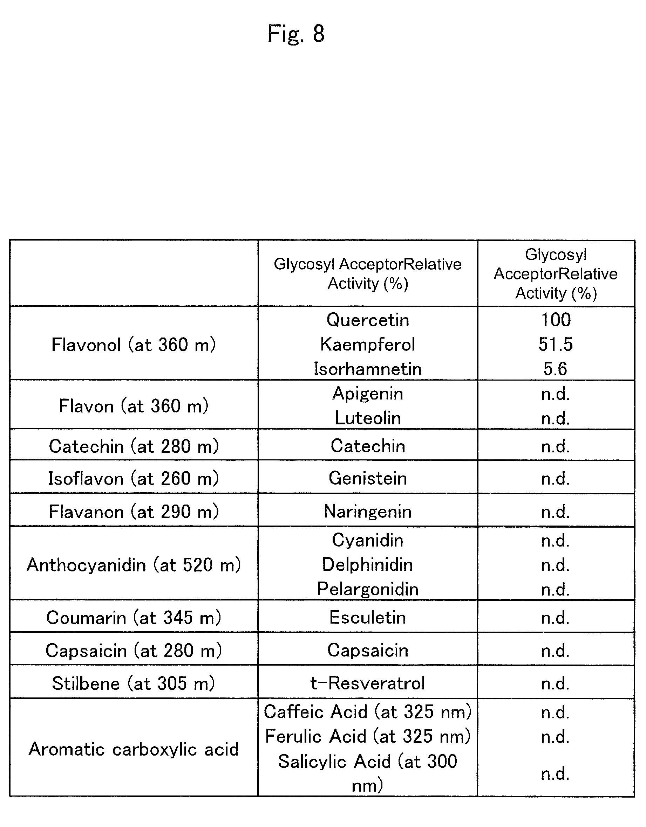

Next, the glycosyl acceptor specificity of this enzyme was examined. As shown in FIG. 8, 100 µM of each glycosyl acceptor, 1 mM UDP-glucuronic acid as a glycosyl donor, 50 mM glycine-NaOH (pH 9.5) buffer, 14 mM 2-mercaptoethanol and 50 ng of the purified VvGT5 described above were reacted at 30°C for 15 minutes. The reaction was started by adding the glycosyl acceptor. The reaction was stopped by adding an equal volume of 40% acetonitrile solution containing 0.1% TFA. After the reaction was stopped, the solution was centrifuged (15000 rpm, 2 mins., 4°C) and 100 µl of the resulting supernatant was provided for the HPLC analysis. The amounts of the products produced were compared for each glycosyl acceptor.

Anthocyanins were analyzed under the conditions below using the HPLC system described above. Conditions for the HPLC analysis of anthocyanins were as follows. Asahipak ODP-50 4E (4.6 x 250 mm, Showa Denko K.K.) as a column was used at room temperature. Moving phase A (0.5% TFA/H₂O) and moving phase B (0.5% TFA/50% GH₃CN) were used. Conditions for elution were as follows: Equilibration was performed with B30% for 3 minutes, followed by linear density gradients (B30%→B65%) for 15 minutes and (B65%→B100%) for further 1 minute, which was maintained for 5 minutes at B100%. Thereafter, the condition was again reverted to B30% to equilibrate for 10 minutes. The flow rate was 0.7 ml/min. Detection was performed at 520 nm. Under the conditions, standard pelargonidin, cyanidin and delphinidin were sequentially eluted in this order at retention times of approximately 25.7 minutes, 23.9 minutes and 20.8 minutes, respectively.

The results of the analysis reveal that VvGT5 acted specifically on flavonols. Among others, VvGT5 showed the highest activity on quercetin (FIG. 8), which coincided with that the main flavonol glucuronide accumulated in grapevine leaves was quercetin 3-glucuronide. Furthermore, this enzyme was subjected to kinetic analysis in a conventional manner; substrate specificities (Km) to quercetin, kaempferol and isorhamnetin were 5.60 ± 1.76 µM, 10.8 ± 0.4 µM and 7.92 ± 0.32 µM, respectively, in this order and catalytic activities (kcat) were 7.16 ± 0.37 s-1, 6.05 ± 0.05 s-1 and 0.413 ± 0.03 s-1, respectively, in this order. As such, it was confirmed also by kinetic parameters that VvGT5 had the highest activity on quercetin.

Next, the glycosyl donor specificity of this enzyme was examined. The reaction of 100 µM quercetin as an acceptor of each sugar, 1mM each glycosyl donor (UDP-glucuronic acid, UDP-glucose or UDP-galactose; all purchased from SIGMA), 50 mM glycine-NaOH (pH 9.5) buffer, 14 mM 2-mercaptoethanol and 50 ng of the purified VvGT5 enzyme described above was carried out at 30°C for 15 minutes. The reaction was started by adding the glycosyl acceptor. The reaction was stopped by adding an equal volume of 40% acetonitrile solution containing 0.1% TFA. After the reaction was stopped, the solution was centrifuged (15000 rpm, 2 mins., 4°C) and 100 µl of the resulting supernatant was provided for the HPLC analysis. The amounts of the products produced in the respective glycosyl donors were compared. As a result, the product was obtained only when UDP-glucuronic acid was used as the substrate, and the substrate specificity (Km) to UDP-glucuronic acid was 17.7 ± 2.9. The results revealed that VvGT5 was specific to UDP-glucuronic acid and was UDP-glucuronic acid-dependent flavonol, 3-O-glucuronosyltransferase (F3GAT).

### REFERENCE EXAMPLE 1

### <Extraction and Purification of Flavonoids from Grapevine>

After 164 g of grapevine leaves (Cabernet Sauvignon cultivar) were ground into powders in liquid nitrogen, the powders were immersed in 1.5 L of 60% CH₃CN and 0.05% HCOOH overnight and then filtered through a celite pad. The filtrate was concentrated under reduced pressure to approximately 1/3 the volume. The concentrate was loaded on 600 ml of CHP-20P. After washing with 600 ml of water, elution was stepwise conducted twice with 300 ml each of 20, 30 and 40% CH₃CN/H₂O. Elution of polyphenols from the 20%-2 fraction was observed. Each fraction from 20%-2 to 40%-l was concentrated and freeze-dried, followed by the HPLC analysis. The yields were 20%-2 (568.6 mg), 30%-1 (345.8 mg), 30%-2 (787.0 mg) and 40%-1 (181.2 mg).

The 30%-1 and 30%-2 fractions where flavonol glycosides were contained in a high purity were purified by preparative HPLC in the two-step procedure described below.

### <Conditions 1 for Preparative HPLC>

Column: Develosil ODS-HG-15/30, φ 50 mm x 500 mm
Moving phase: A-0.05% TFA, B-0.05% TFA/90% CH₃CN
Flow rate: 32 ml/min.
Gradient: B10iso (15 mins.), B15→B40% (100 mins.), B40%iso (30 mins.)
Detection: A350 nm

The flavonol glycosides were confirmed at the elution time of about 103 to 104 minutes. This fraction was concentrated and freeze-dried to give 29.3 mg.

Next, this fraction was again separated under the conditions 2 for preparative HPLC described below.

### <Conditions 2 for Preparative HPLC>

Column: YMC-Pack Polymer C18, φ) 20 mm x 300 mm
Moving phase: A-0.05% TFA, B-0.05% TFA/90% CH₃CN
Flow rate: 6 ml/min.
Gradient: B30iso (20 mins.), B30→B50% (60 mins.), B50%iso (10 mins.)
Detection: A350 nm

Peak A and peak B with the absorption of the flavonol glycosides eluted at 31.01 minutes and 34.03 minutes were observed under the conditions. The fractions of the respective peaks were concentrated and freeze-dried to give 6.5 mg from the peak A fraction and 16.1 mg from the peak B fraction. The peak A coincided with standard quereetin-3-glucoside in retention time. Turning to the peak B, its structure was determined as follows by NMR.

### <Confirmation, etc. of Each Peak by Mass spectrometry (MS) and NMR>

MS was determined on a Q-TOF Premier (manufactured by Micromass, Inc., UK) using ESI equipped with a Z spray ion source in a negative ion V mode. Lock spray mass correction was performed at the collision energy of 45 eV and ion spray voltage of 3 KV. Leucine enkephalin (m/z 554.2777 [M-H]⁻) was used as a reference. As a result, the peak B gave the molecular ion of m/z 477.0664 [M-H]⁻, and the molecular formula was C₂₁H₁₇O₁₃ (err.: -1.0 ppm). The peak A measured under the same conditions gave the Molecular ion of m/z 463.0878 [M-H]⁻, and the molecular formula was C₂₁H₁₉O₁₁ (err.: 0.2 ppm), which coincided with standard quercetin. 3-glucoside as described above and was confirmed to be quercetin 3-glucoside.

The NMR of peak B was determined as follows. After dissolving in (CD3)2SO (DMSO-d6), items of ¹H NMR, ¹³C NMR, ¹H{¹³C}-HSQC, ¹H {¹³C}-HMBC, TOCSY and DQF-COSY were measured on a DMX-500 spectrometer (BRUKER BIOSPIN, Germany) using as internal standards DMSO-d6 ¹H of 2.50 ppm and ¹³C residual peak of δ 39.43.

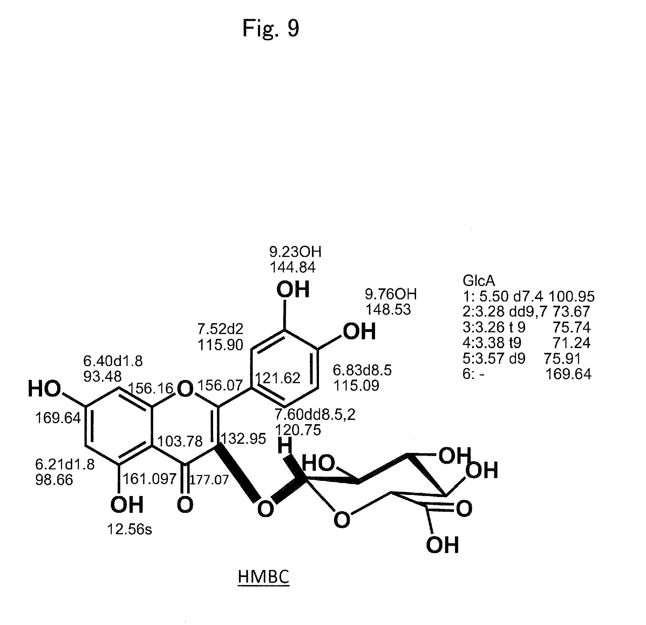

The results of MS and NMR above revealed that the peak B was a quercetin glucuronide and the glucuronic acid was bound to position 3 as the peak from the proton at position 1 of the glucuronide to the carbon at position 3 (δ 132.95) of quercetin was observed in ¹H {¹³C}-HMBC. It was thus confirmed that this compound was quercetin 3-O-glucuronide (FIG. 9, wherein the bold lines denote HMBC). This compound was thus used as standard quercetin 3-O-glucuronide.

### EXAMPLE 3

### Gene Expression Analysis

In order to identify the functional domains of VvGT5, the gene expression pattern of the VvGT5 gene for each grape organ was analyzed by SYBR Green quantitative RT-PCR according to the method described in "Noguchi, A. et al., Plant J., vol. 54, p. 415-427, 2008," using 7500 Real Time PCR System (Applied Biosystems).

The grapevines used were Pinot Noir cultivar and Cabernet Sauvignon cultivar. The total RNA was extracted from each organ (leaves, leafstalks, seeds, fruits, skins) in a manner similar to EXAMPLE 1, and 0.5 µg of the RNA extracted was reverse transcribed (RT) by Random Hexamer Primer, whereby cDNA of each organ was obtained and used as a template for PCR.

As the gene-specific primers used for quantitative PCR, the following 8 primers were designed according to the Primer Express 3.0 Program (Applied Biosystems). The primers shown by SEQ ID NOS: 5 and 6 were used as the VvGT5-specific primers. Specific primers were also designed for anthocyanin 3-glucosyltransferase VvGT1 and VvGT6 homologous to VvGT5 as comparative references, respectively, and provided for the analysis (VvGT1 specific primers: SEQ ID NOS: 7 and 8, VvGT6 specific primers: SEQ ID NOS: 9 and it). Vitis vinifera ubiquitin elongase (GenBank Accession Number: CAO48597) was adopted as the internal control gene and amplified using the gene specific primers (SEQ ID NOS: 11 and 12) described below. The expression level of each VvGT gene was standardized with the internal control gene and the relative expression level was obtained by the ΔCt method (Applied Biosystems).
qVvGT5-Fw1:
   5'-GCT CCA TCT CCT CTG CTC AAA -3'(SEQ ID NO: 5)
qVvGT5-Rv2:
   5'- GAAAGC ACAAGG TCC TCT -3' (SEQ ID NO: 6)
VvGT1-Fw:
   5'- CCC ACC GCC GGT TAT ACC -3'(SEQ ID NO: 7)
VvGT1-Rv:
   5'- CGA CCG AGG TGG GTT TTC T -3' (SEQ ID NO: 8)
VvGT6-Fw:
   5'- GGT TCC CTG GTT GGC AAT TT-3'(SEQ ID NO: 9)
VvGT5-Rv:
   5'- GCA CCC GCC CCA CAA CCT T -3'(SEQ ID NO: 10)
VvUBQ2-Fw:
   5'- TCC AGG ACA AGG AAG GGA TTC -3'(SEQ ID NO: 11)
VvUBQ2-Rv:
   5'- GCC ATC CTC AAG CTG CTT TC -3' (SEQ ID NO: 12)

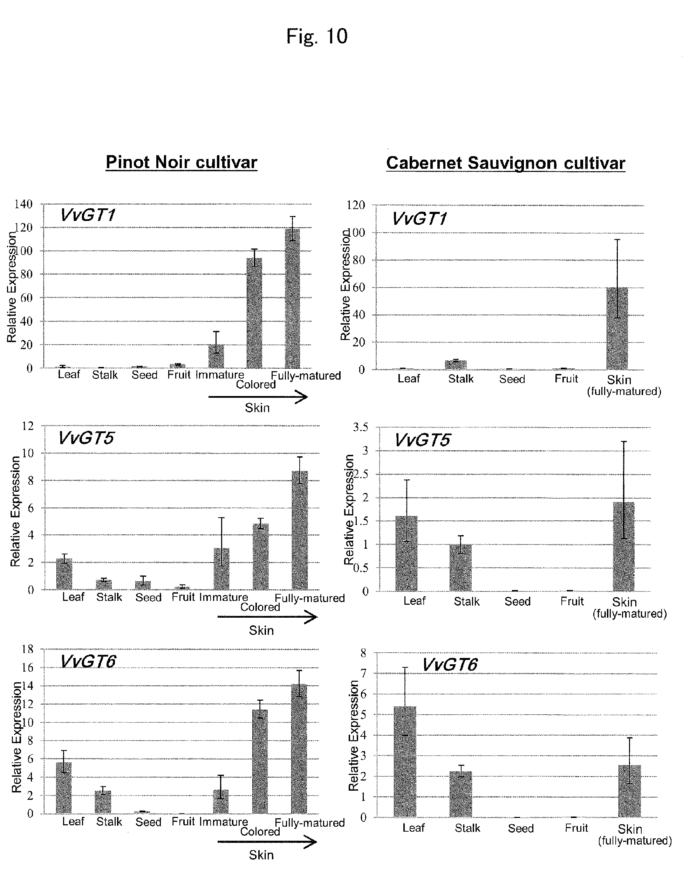

As a result, it was confirmed that the VvGT5 gene was expressed markedly in the leaves where the product was accumulated (FIG. 10). On the other hand, the VvGT1 gene was expressed specifically in the skin, which coincided with the results described in the known literature (Ford, C. M., et al., J. Biol. Chem., vol. 273, p. 9224-9233, 1998). Interestingly, strong expression of the VvGT5 gene was observed also in the skin. The VvGT6 gene showed a gene expression pattern similar to that of the VvGT5 gene. It was thus speculated that location of these genes close together on the genome would affect coordinated gene expression pattern of the two genes (FIG. 2).

### EXAMPLE 4

### Analysis of Flavonoid in Grapevine and Wine

Analysis of the skin flavonoid was performed as it was suggested by the gene expression pattern of VvGT5 in EXAMPLE 3 that flavonol 3-glucuronide would be accumulated also in the skins.

Using the skins of three cultivars of Vitis vinifera (Pinot Noir, Cabernet Sauvignon and Syrah) and the leaves of Pinot Noir and Cabernet Sauvignon as reference controls, 0.8 g fresh weight of each sample was freeze dried. The dried sample was pulverized with a spatula. After 8 ml of 50% acetonitrile solution containing 0.1 % formic acid was added to the sample, the mixture was ultrasonicated at room temperature for 30 minutes to extract flavonoids. The ultrasonicated solution was centrifuged (3000 rpm, 10 minutes, 4°C). The resulting supernatant was filtrated through a filter (Millex LH, pore size of 0.45 µm, manufactured by Millipore, Inc.), which was provided for the HPLC analysis.

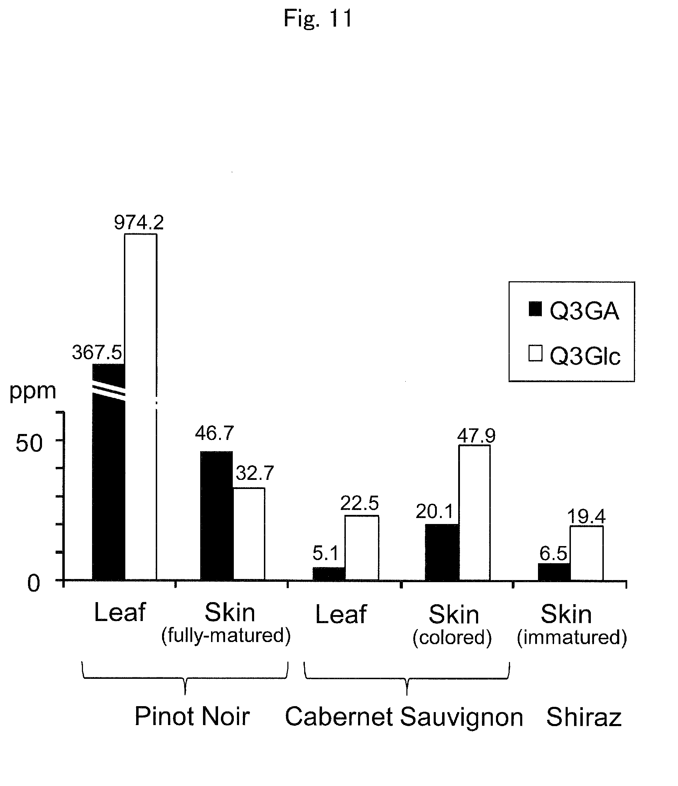

Conditions for the HPLC analysis were as follows. YMC-PAK-Polymer C18 (4.6 mm x 250 mm, YMC) as a column was used at 40°C of a column oven. Moving phase A (0.1% TFA/H₂O) and moving phase B (0.1% TFA/90% CH₃CN) were used. Conditions for elution were as follows: after linear density gradient (B20%→B60%), the system was maintained at 60% for further 10 minutes. Thereafter, it was again reverted to B20% and equilibrated for 10 minutes. The flow rate was 0.8 ml/min. Detection was performed at 350 nm using SPD-M10A Photodiode Array Detector (Shimadzu Corporation). Under the conditions, standard quercetin 3-glucuronide (Q3GA) and quercetin 3-glucoside (Q3Glc) were eluted in this order at retention times of approximately 10.89 minutes and 11.21 minutes, respectively. As a result of the LC analysis, it was confirmed that Q3GA was accumulated in the skin of each grape cultivar (FIG. 11). While the contents were different between the samples, the differences are considered to reflect differences in skin maturity and differences between cultivars.

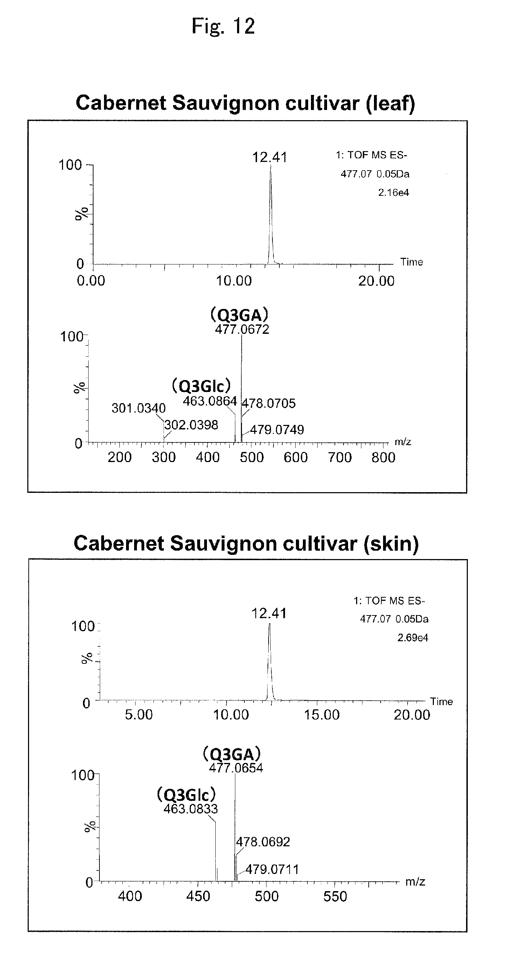

Further in the MS analysis, Q3GA was confirmed in the grape skin (FIG. 12).

Conditions for the LC-MS analysis were as follows. YMC-PAK-Polymer C18 (3.0 mm x 150 mm, YMC) as a column was used. Moving phases used were water containing 0.1% formic acid as solution A and as solution B 100% acetonitrile containing 0.1% formic acid. Elution was performed using linear density gradient for 20 minutes (solution B: 10%→70% (15 minutes)) followed by isocratic elution with solution B 70% for 5 minutes (flow rate: 0.2 ml/min., column oven: 40°C).

Detection was performed by collecting data at 230-500 nm on a photodiode array detector (SPD-M10A, Shimadzu Corporation) and measuring the chromatogram at A350 nm. Also, Q-TOF Premier (manufactured by Micromass, Inc., UK) was connected to the PDA detector and the molecular weight of the product was measured under the following conditions. Conditions for the MS measurement were set in a negative ion V mode (ionization: ESI, lock spray reference: leucine enkephalin, Capillary: 2.6 kV, Cone: 35V, collision energy: 5eV).

Under the conditions, Q3Glc was eluted in R.T. of 12.32 minutes and gave the molecular ion of 463.09 [M-H]⁻, and -3GlcA was eluted in R.T. of 12.41 minutes and gave the molecular ion of 477.07 [M-H]⁻. The peak of Q3GA in the leaf and skin was confirmed by the mass chromatogram.

Next, Q3GA was searched in the foodstuff, in which grapevine was used as a raw material.

Red wine (Beaujolais 2007, Gamay cultivar), red wine (Bourgogne 2006, Pinot Noir cultivar) and raisin (dried grape, Chinese-grown white grape) were examined.

As for wine, an equal volume of water was added to 10 ml of wine and the resulting solution was adsorbed onto a SEP-PAK 1 cc cartridge (Waters, Inc.). After washing with 10 ml of water, elution was performed by 1 ml of 90% acetonitrile to give a 10-fold concentrated sample.

As for raisin, 10 g was frozen in liquid nitrogen and pulverized in a mortar, followed by extraction with 20 ml of 50% acetonitrile. After 85 ml of water was added to 15 ml of the extract, the mixture was adsorbed onto a SEP-PAK 20 cc cartridge (Waters, Inc.). The mixture was washed with 100 ml of water and then eluted with 2 ml of 90% acetonitrile to give a LC-MS sample solution.

Conditions for LC-MS were as follows. YMC-Pack Polymer C18 column (YMC, 2.0 mm x 150 mm, 6 µm) was used. For the moving phase, water containing 0.1 % formic acid was used as solution A and 90% acetonitrile containing 0.1% formic acid was used as solution B. Elution was performed using linear density gradient for 15 minutes (solution A 80% : solution B 20% → solution A 40%: solution B 60%) followed by isocratic elution with solution A 40% : solution B 60% for 5 minutes (flow rate: 0.2 ml/min.).

Detection was performed as follows. Data were collected at 230-500 nm on a photodiode array detector (SPD-M10A, Shimadzu Corporation). Also, TOF-MS Detector (LCMS-IT-TOF, manufactured by Shimadzu Corporation) was connected to the PDA detector and the molecular weight of the product was measured. Conditions for MS were set in both negative and positive ion modes; the molecular weight measured ranged from 100 to 1000Da and interference voltages of 4.5 kV and -3.5kV, respectively.

Under the conditions, standard Q3Glc and Q3GA were eluted at approximately 8.5 minutes. The three food samples described above were investigated at about 8.5 minutes. In all the samples, the molecular ion of m/z 477.04 [M-H]⁻, which coincided with Q3GA, was detected in the negative ion mode. At the same time, the molecular ion of m/z 463.06 [M-H]-, which coincided with Q3Glc, was detected in the negative ion mode.

As indicated from the gene expression pattern of VvGT5, it was confirmed that quercetin 3-glucuronide was present in the grape skin and foods containing the same (wine and raisin). It is thus shown that VvGT5 was expressed mainly in the grape leaf and skin and glucuronidation occurred at the position 3 of flavonols.

### EXAMPLE 5

### Point Mutant Analysis of VvGT5 on Glycosyl Donor Selectivity

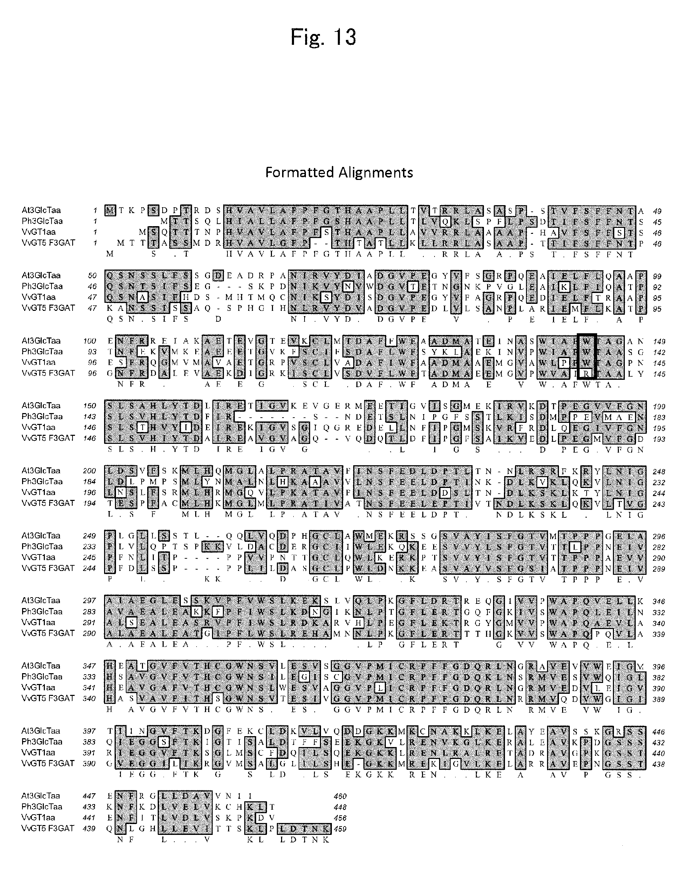

As shown in EXAMPLE 2, it became clear that VvGT5 utilized UDP-glucuronide specifically. To clarify the amino acid residue to determine this glycosyl donor selectivity, multiple alignment of amino acid sequence was performed using Clustal W (FIG. 13). As a result, it was found that arginine (R) was substituted in VvGT5 for tryptophan (W) present around the amino acid 140 from the N terminus, which was highly conserved in anthocyanin 3-O-glucosyltransferase VvGT 1 of Vitis vinifera having high homology to VvGT5 and in flavonoid 3-O-glucosyltransferases At3GlcT (UGT78D2; GenBank Accession Number: AAM91139) and Ph3GlcT (GenBank Accession Number: BAA89008) from Arabidopsis thaliana and petunia, respectively. It is known from the analysis of crystal structure that threonine (T) in the vicinity of the C terminal side of these amino acid residues interacts with UDP-glucose as a glycosyl donor (Often, W. et al., EMBO J., vol. 25, p. 1396-1405, 2006). It is then indicated that the arginine residue (amino acid residue at position 140 in the amino acid sequence of SEQ ID NO: 4) specifically found in VvGT5 would somehow interact with UDP-glucuronic acid as its glycosyl donor.

In order to elucidate the function of this unique arginine residue in glycosyl donor selectivity, the VvGT5-R14OW mutant wherein the arginine residue at position 140 of VvGT5 is substituted with tryptophan residue (the notation "R140" designates mutation that the arginine residue (R) at position 140 is substituted with tryptophan (W) residue) was prepared using Quick Change 11 Site-Directed Mutagenesis Kit (Strata gene Corp.) according to the protocol recommended by the kit, in which pET 15b vector inserted with the aforesaid wild VvGT5 was used as a vector and the synthetic oligoprimers (SEQ ID NOS: 13 and 14) were used.

PCR conditions: The reaction consisting of one cycle of thermal denaturation at 96°C for 30 seconds → 58°C for 1 minute → 68°C for 7 minutes was repeated 16 cycles. Restriction enzyme Dpn I was added to the PCR solution. After the mixture was treated at 37°C for an hour, 10 µl of the reaction solution was transformed to Escherichia coli (Mach strain). The resulting plasmid was recovered. It was confirmed by sequencing that the target mutagenesis occurred.
VvGT5 R140W Quik Fw:
   5'- GGGTGGCAATTTGGACTGCTG -3'(SEQ ID NO: 13)
VvGT5 R140W Quik Rv:
   5'-CAGCAGTCCAAATTGC ACCC -3'(SEQ ID NO: 14)

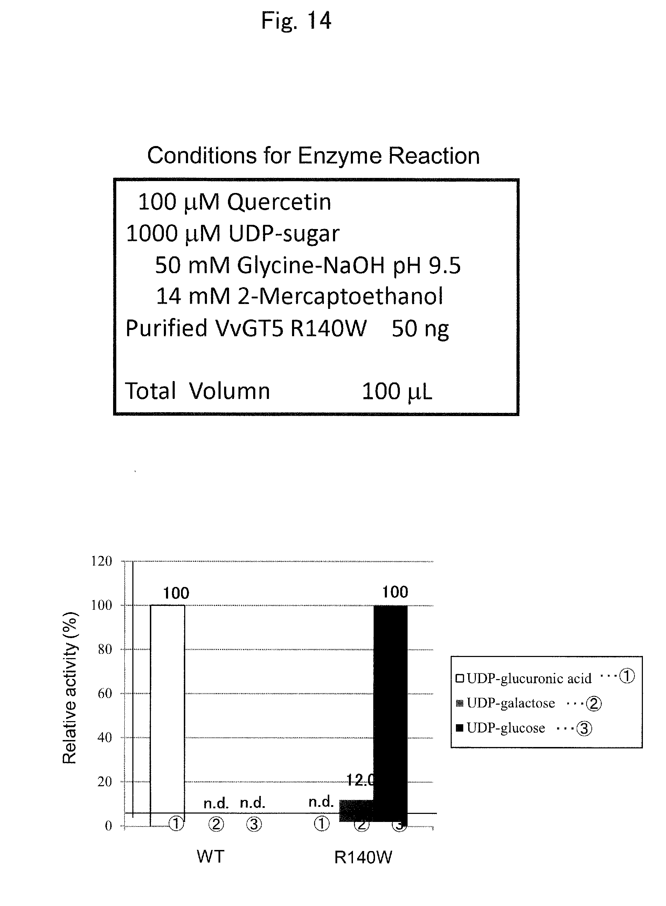

The VvGT5-R140W mutant prepared was transformed to the Escherichia coli BL21 strain in a manner similar to EXAMPLE 2. After addition of 1 mM IPTG, the transformant was incubated at 18°C for 20 hours to prepare as His-tagged fusion VvGT5-R140W protein. The glycosyl donor selectivity was examined in a manner similar to EXAMPLE 2. The results indicate that UDP-glucuronic acid was not used as a substrate in the VvGTS-R140W mutant but instead the main glycosyl donor was UDP-glucose(FIG. 14).

The foregoing shows that the arginine residue at position 140 of VvGT5 portrays a crucial role for the recognition of UDP-glucuronic acid. It was speculated from the foregoing that an electric interaction between the guanidinium group which is a side chain characteristic of the arginine residue and the carboxylic acid which is a functional group characteristic of glucuronic acid would be important for the recognition of UDP-glucuronic acid. It was thus considered that such an arginine residue as R 140 of VvGT5 would probably be carried on the UGT enzyme where UDP-glucuronic acid is a specific glycosyl donor.

### INDUSTRIAL APPLICABILITY

As described above, the UGT enzyme (VvF3GAT) having a novel activity of transferring glucuronic acid from Vitis vinifera to the position 3 of flavonoids could be isolated. Furthermore, the amino acid residue which determines the specificity to the UDP-glucuronic acid could also be identified. By using the present invention, the position 3 of flavonoids can be glucuronidated artificially. Accordingly, the invention can contribute to the development of new functional food materials and the development of plants capable of producing useful compounds. Therefore, the present invention is extremely useful for a wide variety of applications including agriculture, food industry, drug industry and industries related thereto.

### Sequence Listing Free Text

SEQ ID NO: 1: synthetic DNA
SEQ ID NO: 2: synthetic DNA
SEQ ID NO: 5: synthetic DNA
SEQ ID NO: 6: synthetic DNA
SEQ ID NO: 7: synthetic DNA
SEQ ID NO: 8: synthetic DNA
SEQ ID NO: 9: synthetic DNA
SEQ ID NO: 10: synthetic DNA
SEQ ID NO: 11: synthetic DNA
SEQ ID NO: 12: synthetic DNA
SEQ ID NO: 13: synthetic DNA
SEQ ID NO: 14: synthetic DNA

## Claims

1. A polynucleotide of any one of (a) to (f) below:
(a) a polynucleotide comprising a polynucleotide consisting of the nucleotide sequence represented by SEQ ID NO: 3;
(b) a polynucleotide comprising a polynucleotide encoding a protein consisting of the amino acid sequence represented by SEQ ID NO: 4;
(c) a polynucleotide comprising a polynucleotide encoding a protein consisting of the amino acid sequence wherein 1 to 15 amino acids are deleted, substituted, inserted and/or added in the amino acid sequence represented by SEQ ID NO: 4, and having a UDP-glucuronosyltransferase activity;
(d) a polynucleotide comprising a polynucleotide encoding a protein that has an amino acid sequence having a homology of at least 80% to the amino acid sequence represented by SEQ ID NO: 4, and has a UDP-glucuronosyltransferase activity;
(e) a polynucleotide comprising a polynucleotide encoding a protein that hybridizes under stringent conditions with a polynucleotide consisting of a nucleotide sequence complementary to the nucleotide sequence represented by SEQ ID NO: 3 and has a UDP-glucuronosyltransferase activity; or,
(f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4, and has a UDP-glucuronosyltransferase activity

2. A polynucleotide of any one of (g) to (j) below:
(g) a polynucleotide comprising a polynucleotide encoding a protein consisting of the amino acid sequence wherein not greater than 10 amino acids are deleted, substituted, inserted and/or added in the amino acid sequence represented by SEQ ID NO: 4, and having a UDP-glucuronosyltransfierase activity;
(h) a polynucleotide comprising a polynucleotide encoding a protein that has an amino acid sequence having a homology of at least 90% to the amino acid sequence represented by SEQ ID NO: 4, and has a UDP-glucuronosyltransferase activity;
(i) a polynucleotide comprising a polynucleotide encoding a protein that hybridizes under highly stringent conditions with a polynucleotide consisting of a nucleotide sequence complementary to the nucleotide sequence represented by SEQ ID NO: 3, and has a UDP-glucuronosyltransferase activity; or,
(j) a polynucleotide comprising a polynucleotide encoding a protein that hybridizes under highly stringent conditions with a polynucleotide consisting of a nucleotide sequence complementary to the nucleotide sequence of a polynucleotide encoding a protein consisting of the amino acid sequence represented by SEQ ID NO: 4, and has a UDP-glucuronosyltransferase activity.

3. The polynucleotide according to claim 1 or 2, wherein the polynucleotide of(c) to (j) above are the polynucleotide of(c') to (j') below, respectively:
(c') a polynucleotide comprising a polynucleotide encoding a protein consisting of the amino acid sequence wherein 1 to 15 amino acids except for the amino acid at position 140 are deleted, substituted, inserted and/or added in the amino acid sequence represented by SEQ ID NO: 4, and having a UDP-glucuronosyltransferase activity;
(d') a polynucleotide comprising a polynucleotide encoding a protein that has an amino acid sequence having a homology of at least 80% to the amino acid sequence represented by SEQ ID NO: 4 wherein the amino acid corresponding to the amino acid at position 140 in the amino acid sequence represented by SEQ ID NO: 4 is arginine, and has a UDP-glucuronosyltransferase activity;
(e') a polynucleotide comprising a polynucleotide encoding a protein that hybridizes under stringent conditions with a polynucleotide consisting of a nucleotide sequence complementary to the nucleotide sequence represented by SEQ ID NO: 3 wherein the nucleotides corresponding to the nucleotides at positions 418-420 in the nucleotide sequence represented by SEQ ID NO: 3 are the nucleotides encoding arginine, and has a UDP-glucuronosyltransferase activity;
(f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4 wherein the nucleotides corresponding to the nucleotides at positions 418-420 in the nucleotide sequence of a polynucleotide encoding a protein consisting of the amino acid sequence represented by SEQ ID NO: 4 are the nucleotides encoding arginine, and has a UDP-glucuronosyltransferase activity;
(g') a polynucleotide comprising a polynucleotide encoding a protein consisting of the amino acid sequence wherein not greater than 10 amino acids except for the amino acid at position 140 are deleted, substituted, inserted and/or added in the amino acid sequence represented by SEQ ID NO: 4, and having a UDP-glucuronosyltransferase activity;
(h') a polynucleotide comprising a polynucleotide encoding a protein that has an amino acid sequence having a homology of at least 90% to the amino acid sequence represented by SEQ ID NO: 4 wherein the amino acid corresponding to the amino acid at position 140 in the amino acid sequence represented by SEQ ID NO: 4 is arginine, and has a UDP-glucuronosyltransferase activity;
(i') a polynucleotide comprising a polynucleotide encoding a protein that hybridizes under highly stringent conditions with a polynucleotide consisting of a nucleotide sequence complementary to the nucleotide sequence represented by SEQ ID NO: 3 wherein the nucleotides corresponding to the nucleotides at positions 418-420 in the nucleotide sequence represented by SEQ ID NO: 3 are the nucleotides encoding arginine, and has a UDP-glucuronosyltransferase activity; or,
(j') a polynucleotide comprising a polynucleotide encoding a protein that hybridizes under highly stringent conditions with a polynucleotide consisting of a nucleotide sequence complementary to the nucleotide sequence of a polynucleotide encoding a protein consisting of the amino acid sequence represented by SEQ ID NO: 4 wherein the nucleotides corresponding to the nucleotides at positions 418-420 in the nucleotide sequence of a polynucleotide encoding a protein consisting of the amino acid sequence represented by SEQ ID NO: 4 are the nucleotides encoding arginine, and has a UDP-glucuronosyltransferase activity.

4. The polynucleotide according to any one of claims 1 to 3, wherein the UDP-glucuranosyltransferase activity is a flavonoid 3-O-glucnronosyltransferase activity.

5. The polynucleotide according to claim 1, which comprises a polynucleotide consisting of the nucleotide sequence represented by SEQ ID NO: 3.

6. The polynucleotide according to claim 1, which comprises a polynucleotide encoding a protein consisting of the amino acid sequence represented by SEQ ID NO: 4.

7. The polynucleotide according to any one of claims 1 to 6, which is a DNA.

8. A protein encoded by the polynucleotide according to any one of claims 1 to 7.

9. A vector comprising the polynucleotide according to any one of claims 1 to 7.

10. A transformant, wherein the polynucleotide according to any one of claims 1 to 7 is introduced.

11. A transformant, wherein the vector according to claim 9 is introduced.

12. A method for producing the protein according to claim 8, which comprises using the transformant according to claim 10 or 11.

13. A method for producing a glucuronate conjugate, which comprises forming the glucuronate conjugate from UDP-glucuronic acid and a glycosyl acceptor substrate using the protein according to claim 8 as a catalyst.

14. The method according to claim 13, herein the glycosyl acceptor substrate is a flavonoid.
